# EUROPÄISCHE PATENTSCHRIFT

(11) **EP 0 765 919 B1**
(45) Veröffentlichungstag und Bekanntmachung des Hinweises auf die Patenterteilung: **21.02.2001**
(21) Anmeldenummer: 96810617.9
(22) Anmeldetag: 17.09.1996
(51) Int. Cl.: C09B 67/48, C09B 67/22, C08K 5/3415

(54) **Monophasige feste Lösungen mit asymmetrischen Pyrrolo-[4,3-c]-pyrrolen als Wirten**
Monophasic solid solutions with asymmetrical pyrrolo-(4-3-c)-pyrroles as host
Solutions solides monophasiques avec des pyrrolo-(4-3-c)pyrroles asymétriques

(30) Priorität: 26.09.1995 CH 271995
(43) Veröffentlichungstag der Anmeldung: 02.04.1997
(73) Patentinhaber: Ciba Specialty Chemicals Holding Inc., 4057 Basel (CH)
(72) Erfinder: Hao, Zhimin, 1723 Marly (CH); Iqbal, Abul, 1732 Arconciel (CH)

(56) Entgegenhaltungen:
- EP-A- 0 094 911
- EP-A- 0 181 290
- EP-A- 0 256 983
- EP-A- 0 277 914
- EP-A- 0 654 506
- EP-A- 0 704 497
- EP-A- 0 707 049

## Beschreibung

Die vorliegende Erfindung betrifft neue, einphasige feste Lösungen aus einem substituierten, asymmetrischen 2,5-Dihydro-1,4-diketopyrrolopyrrol und einem 2,5-Dihydro-1,4-diketopyrrolopyrrol oder einem Chinacridon, sowie ihre Verwendung als Pigmente.

2,5-Dihydro-1,4-diketopyrrolopyrrole (DPP), darunter auch asymmetrische des Typs

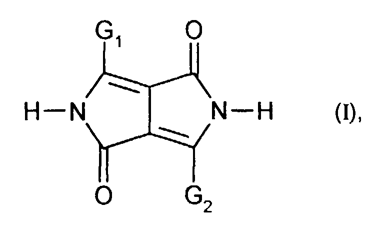

ihre Herstellung und ihre Verwendung als Pigmente, sind zum Beispiel in US-4,579,949 beschrieben. Solche DPP sind auch als Bestandteile von festen Lösungen aus US-4,783,540 und US-4,810,304 bekannt.

US-4,783,540 beschreibt, dass beim Mischen zweier verschiedenen 2,5-Dihydro-1,4-diketopyrrolopyrrole und nachfolgender Behandlung durch Kneten, Mahlen oder Umfällen, feste Lösungen erhalten werden können. Als Beispiele asymmetrischer DPP der Formel (I) sind 3-Phenyl-6-(3-chlor-phenyl)-2,5-dihydro-1,4-diketopyrrolopyrrol und 3-Phenyl-6-(4-chlor-phenyl)-2,5-dihydro-1,4-diketopyrrolopyrrol veranschaulicht, welche zusammen mit symmetrischen DPP verwendet werden [darunter auch solche der Formel (IIa), worin G₃ und G₄ gleich sind].

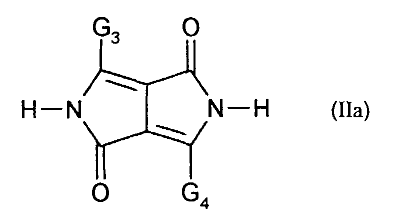

US-4,810,304 beschreibt, dass beim Mischen eines 2,5-Dihydro-1,4-diketopyrrolopyrrols und eines Chinacridons [darunter auch solche der Formel (IIb)] und nachfolgender

Behandlung durch Kneten, Mahlen oder Umfällen, ebenfalls feste Lösungen erhalten werden können. Es sind keine Beispiele mit asymmetrischen DPP der Formel (I) als Komponenten angegeben.

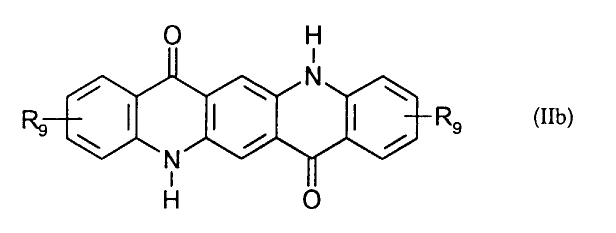

Die Röntgenbeugungsdiagramme dieser festen Lösungen sind in beiden Fällen von der Summe der Röntgenbeugungsdiagramme der Einzelkomponenten unterschiedlich. Bei allen nach den offenbarten Beispielen erhältlichen Produkten unterscheiden sie sich allerdings auch deutlich von den Röntgenbeugungsdiagrammen der reinen kristallinen Einzelkomponenten. Es handelt sich also dabei ausschliesslich um mehrphasige feste Lösungen, welche kein präzises einheitliches Kristallgitter aufweisen. Zudem fallen diese Produkte in unerwünschter, weitgehend amorpher Form an.

Es hat sich zudem herausgestellt, dass solche mehrphasigen festen Lösungen den gewünschten Eigenschaften, wie beispielsweise hohe Licht- und Wetterstabilität und genau reproduzierbare Nuance, nicht im gewünschten Mass genügen.

Es ist nun gefunden worden, dass man einphasige feste Lösungen mit überraschend hoher Licht- und Wetterstabilität und genau reproduzierbarer Nuance bekommt, wenn man als Wirt ein asymmetrisches 2,5-Dihydro-1,4-diketopyrrolopyrrol der Formel (I) mit bestimmten Gruppen G₁ und G₂, und als Gast in bestimmten molaren Verhältnissen ein 2,5-Dihydro-1,4-diketopyrrolopyrrol der Formel (IIa) mit bestimmten Gruppen G₃ und G₄ oder ein Chinacridon der Formel (IIb) mit bestimmten Substituenten R₉ verwendet. Die Kristallinität dieser einphasigen festen Lösungen ist ausgezeichnet, charakterisiert durch Glanzwinkel-Linien mit einer Halbwertsbreite ≤ 1,0 2Θ bevorzugt ≤ 0,8 2Θ, in deren Röntgenbeugungsdiagramm. Das Röntgenbeugungsdiagramm der einphasigen festen Lösung ist jeweils mit demjenigen des reinen asymmetrischen 2,5-Dihydro-1,4-diketopyrrolopyrrols der Formel (I) fast identisch, das heisst der Gast gibt jeweils seine kristalline Struktur gänzlich auf und die erhaltene einphasige feste Lösung ist mit dem Wirt isomorph. Es liegen auch keine Mischkristalle vor.

In der Literatur sind die von verschiedenen Autoren wie G. H. Van't Hoff, A.I. Kitaigorodsky und A. Whitacker verwendeten Definitionen für feste Lösungen und Mischkristalle oft widersprüchlich (vgl. z.B. "Analytical chemistry of synthetic dyes", Kapitel 10 / Seite 269, Herausgeber K. Venkataraman, Verlag J. Wiley, New York 1977).

Was hier unter "einphasige feste Lösung", "mehrphasige feste Lösung" oder "Mischkristall" zu verstehen ist, soll deshalb den folgenden neuen Definitionen, welche den heutigen verbesserten Kenntnissen solcher Systeme angepasst wurden, entnommen werden:
- Eine *mehrphasige feste Lösung* besitzt kein präzises einheitliches Kristallgitter. Sie unterscheidet sich von einem physikalischen Gemisch ihrer einzelnen Komponenten dadurch, dass das Kristallgitter mindestens einer ihrer Komponenten teilweise oder vollständig verändert ist. Die Signale im Röntgenbeugungsdiagramm sind im Vergleich zum physikalischen Gemisch der Komponenten verbreitert, verschoben oder in der Intensität verändert. Unterschiedliche Proportionen der Komponenten ergeben in der Regel unterschiedliche Resultate.
- Eine *einphasige (oder monophasige) feste Lösung* besitzt ein Kristallgitter, welches mit dem Kristallgitter einer ihrer Komponenten identisch ist. Eine Komponente liegt als "Gast" eingebettet im Kristallgitter der als "Wirt" fungierenden anderen Komponente. Unterschiedliche Proportionen der Komponenten ergeben innerhalb gewisser Grenzen fast identische Resultate.
- Ein *Mischkristall* besitzt eine präzise Zusammensetzung sowie ein einheitliches Kristallgitter, welches von den Kristallgittern aller seiner Komponenten unterschiedlich ist. Führen unterschiedliche Proportionen der Komponenten innerhalb gewisser Grenzen zum gleichen Resultat, so liegt eine feste Lösung vor, worin der Mischkristall als Wirt fungiert.

Um jegliches Missverständnis auszuschliessen, sei noch darauf hingewiesen, dass es unter anderen auch amorphe Gebilde und aus verschiedenen Partikeln unterschiedlicher physikalischer Natur bestehende gemischte Aggregate geben kann, wie beispielsweise ein Aggregat aus verschiedenen Komponenten in jeweils reiner Kristallmodifikation. Solche amorphen Gebilde und gemischten Aggregate dürfen weder festen Lösungen noch Mischkristallen gleichgesetzt werden und besitzen andere grundsätzliche Eigenschaften.

Die vorliegende Erfindung betrifft demnach eine feste Lösung, bestehend aus 60-90 mol-% eines asymmetrischen 2,5-Dihydro-1,4-diketopyrrolo[3,4-c]pyrrols der Formel (I) 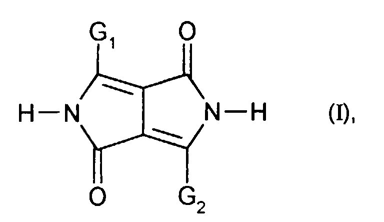 worin G₁ und G₂ unabhängig voneinander unterschiedliche Reste 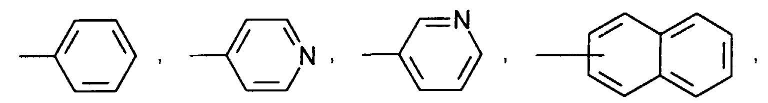 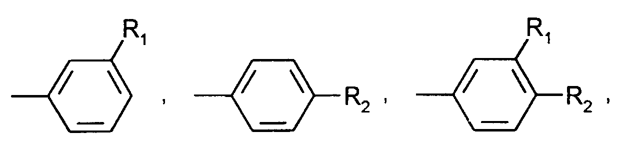 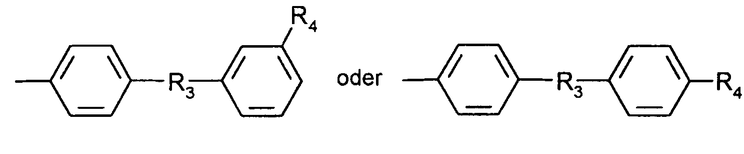 sind,
worin
- R₁ und R₂: unabhängig voneinander Fluor, Chlor, Brom, Cyano, Nitro, Trifluormethyl, C₁-C₈-Alkyl, C₅-C₆-Cycloalkyl, Phenyl, C₁-C₈-Alkoxy, C₁-C₈-Alkoxycarbonyl, C₁-C₈-Alkylamino, C₁-C₈-Alkylaminocarbonyl, C₁-C₈-Dialkylamino, C₁-C₈-Dialkylaminocarbonyl oder Morpholino,
- R₃: -O-, -NR₅-, -N=N- oder -SO₂-,
- R₄: Wasserstoff, Fluor, Chlor, Brom, Cyano, Nitro, Trifluormethyl, C₁-C₈-Alkyl, C₁-C₈-Alkoxy, C₁-C₈-Alkylamino, C₁-C₈-Dialkylamino, C₁-C₈-Alkoxycarbonyl, C₁-C₈-Alkylaminocarbonyl oder C₁-C₈-Dialkylaminocarbonyl, und
- R₅: Wasserstoff, Methyl oder Ethyl bedeuten,
jedoch wenn einer der Reste G₁ oder G₂ 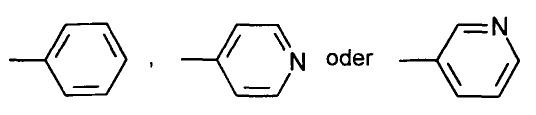 ist,
der jeweils andere Rest G₂ oder G₁ nicht 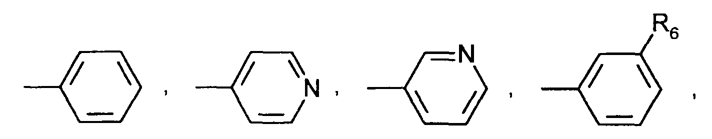 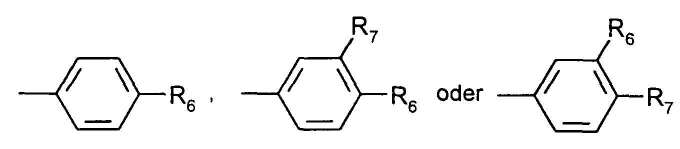 ist,
worin
R₆ Chlor und R₇Fluor oder Trifluormethyl sind, oder R₆ sowie R₇ unabhängig voneinander Fluor, Methyl oder Trifluormethyl sind;
und 40-10 mol-% eines 2,5-Dihydro-1,4-diketopyrrolo[3,4-c]pyrrols der Formel (IIa) 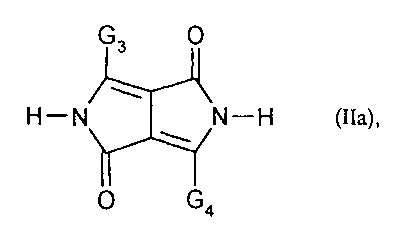 worin G₃ und G₄ unabhängig voneinander gleiche oder verschiedene Reste 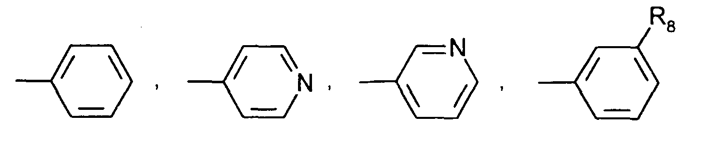 oder 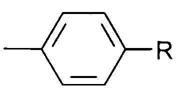 sind,
worin
- R₈: Fluor, Chlor, Cyano, Nitro, Trifluormethyl, C₁-C₄-Alkyl, C₁-C₄-Alkoxy, C₁-C₄-Alkylamino oder C₁-C₄-Dialkylamino bedeutet;
oder 40-10 mol-% eines Chinacridons der Formel (IIb) 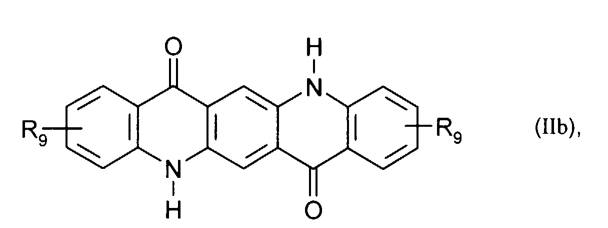 worin
- R₉: Wasserstoff, Halogen, C₁-C₄-Alkyl oder C₁-C₄-Alkoxy bedeutet;
dadurch gekennzeichnet, dass es sich dabei um eine einphasige feste Lösung handelt, welche die Kristallstruktur des asymmetrischen 2,5-Dihydro-1,4-diketopyrrolo[3,4-c]pyrrols der Formel (I) aufweist.

C₁-C₈-Alkyl steht z.B. für Methyl, Ethyl, n-Propyl, Isopropyl, n-Butyl, sec.-Butyl, tert.-Butyl, n-Amyl, tert.-Amyl, n-Hexyl, n-Heptyl, n-Octyl, 2-Ethylhexyl oder 2,4,4-Trimethyl-2-pentyl.

C₁-C₈-Alkoxy steht, auch in C₁-C₈-Alkoxycarbonyl, für -O-C₁-C₈-Alkyl, wie Methoxy, Ethoxy, n-Propoxy, Isopropoxy, Butyloxy, Hexyloxy oder Octyloxy.

C₁-C₈-Alkylamino steht, auch in C₁-C₈-Alkylaminocarbonyl, für -NH-C₁-C₈-Alkyl, wie Methylamino, Ethylamino, Propylamino, Hexylamino oder Octylamino.

C₂-C₈-Dialkylamino steht, auch in C₂-C₈-Dialkylaminocarbonyl, für 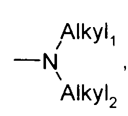 worin die Anzahl Kohlenstoffatome in beiden Alkylgruppen total 2 bis 8 beträgt, wie Dimethylamino, Diethylamino, Dipropylamino, Dibutylamino, Methylhexylamino oder Ethylhexylamino.

C₅-C₆-Cycloalkyl steht z.B. für Cyclopentyl und insbesondere für Cyclohexyl.

Bevorzugt sind asymmetrische 2,5-Dihydro-1,4-diketopyrrolo[3,4-c]pyrrole der Formel (I) 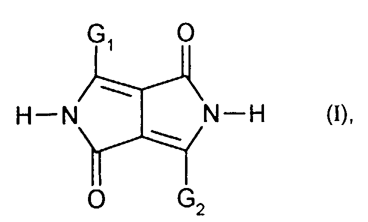 worin
- G₁ und G₂: unabhängig voneinander unterschiedliche Reste 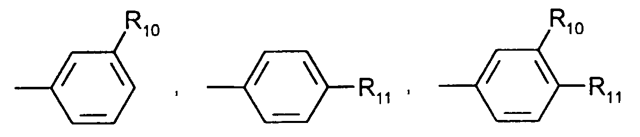
oder 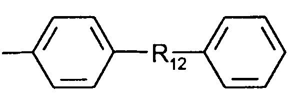 sind,
worin
- R₁₀ und R₁₁: unabhängig voneinander Chlor, Brom, Cyano, Nitro, Trifluormethyl, C₁-C₈-Alkyl, Phenyl, C₁-C₈-Alkoxy, C₁-C₈-Alkoxycarbonyl, C₁-C₈-Alkylamino oder C₁-C₈-Dialkylamino sind,
- und R₁₂: -NR₅- oder -SO₂- bedeutet.

Als Beispiele bevorzugter asymmetrischer 2,5-Dihydro-1,4-diketopyrrolo[3,4-c]pyrrole der Formel (I) seien insbesondere genannt:
- 3-(4'-Chlor-phenyl)-6-(3'-methyl-phenyl)-2,5-dihydro-1,4-diketopyrrolo[3,4-c]pyrrol,
- 3-(4'-Chlor-phenyl)-6-(4'-methyl-phenyl)-2,5-dihydro-1,4-diketopyrrolo[3,4-c]pyrrol,
- 3-Phenyl-6-(4'-biphenylyl)-2,5-dihydro-1,4-diketopyrrolo[3,4-c]pyrrol,
- 3-Phenyl-6-(4'-tert-butyl-phenyl)-2,5-dihydro-1,4-diketopyrrolo[3,4-c]pyrrol,
- 3-Phenyl-6-(3',4'-dichlor-phenyl)-2,5-dihydro-1,4-diketopyrrolo[3,4-c]pyrrol,
- 3-(4'-Chlor-phenyl)-6-(4'-biphenylyl)-2,5-dihydro-1,4-diketopyrrolo[3,4-c]pyrrol,
- 3-(4'-Chlor-phenyl)-6-(4'-tert-butyl-phenyl)-2,5-dihydro-1,4-diketopyrrolo[3,4-c]pyrrol,
- 3-(4'-Chlor-phenyl)-6-(3'-methyl-phenyl)-2,5-dihydro-1,4-diketopyrrolo[3,4-c]pyrrol und
- 3-(4'-Chlor-phenyl)-6-(3'-cyano-phenyl)-2,5-dihydro-1,4-diketopyrrolo[3,4-c]pyrrol.

Besonders bevorzugt sind asymmetrische 2,5-Dihydro-1,4-diketopyrrolo[3,4-c]pyrrole der Formel (I) 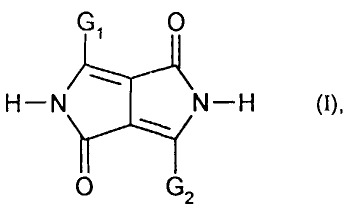 worin
- G₁: ein Rest 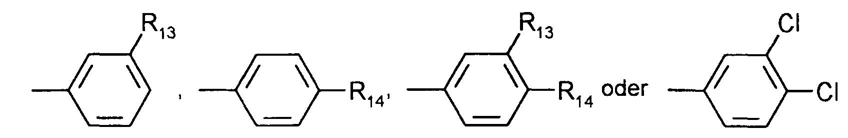 ist,
worin R₁₃ und R₁₄ Nitro, C₄-C₈-Alkyl, Phenyl, C₄-C₈-Alkoxy oder C₄-C₈-Dialkylamino, insbesondere tert.-Butyl oder Phenyl, bedeuten, wobei G₂ einen von G₁ unterschiedlichen, der ganz oben angegebenen Definition entsprechenden Rest ist.

Diese besonders bevorzugten asymmetrischen 2,5-Dihydro-1,4-diketopyrrolo[3,4-c]pyrrole der Formel (I) erweisen sich besonders überraschend auch dann als zur Bildung von monophasigen festen Lösungen geeignete Wirte, wenn G₂ unsubstituiertes Phenyl ist.

Als Beispiele besonders bevorzugter asymmetrischer 2,5-Dihydro-1,4-diketopyrrolo[3,4-c]-pyrrole der Formel (I) seien insbesondere genannt:
- 3-Phenyl-6-(4'-biphenylyl)-2,5-dihydro-1,4-diketopyrrolo[3,4-c]pyrrol,
- 3-Phenyl-6-(4'-tert-butyl-phenyl)-2,5-dihydro-1,4-diketopyrrolo[3,4-c]pyrrol und
- 3-Phenyl-6-(3',4'-dichlor-phenyl)-2,5-dihydro-1,4-diketopyrrolo[3,4-c]pyrrol.

Ganz besonders bevorzugt sind asymmetrische 2,5-Dihydro-1,4-diketopyrrolo[3,4-c]pyrrole der Formel (I) 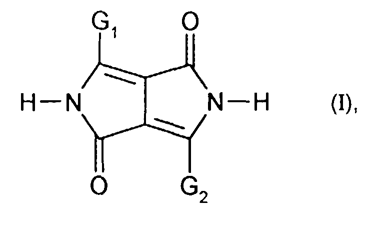 worin G₁ ein Rest 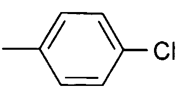 und G₂ ein unterschiedlicher Rest 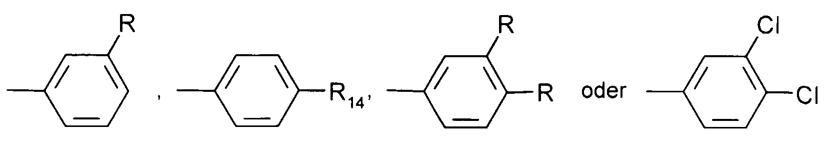 sind
worin R₁₃ und R₁₄ Nitro, C₄-C₈-Alkyl, Phenyl, C₄-C₈-Alkoxy oder C₄-C₈-Dialkylamino, insbesondere tert.-Butyl oder Phenyl, bedeuten.

Als Beispiele ganz besonders bevorzugter asymmetrischer 2,5-Dihydro-1,4-diketopyrrolo-[3,4-c]pyrrole der Formel (I) seien insbesondere genannt:
- 3-(4'-Chlor-phenyl)-6-(4'-biphenylyl)-2,5-dihydro-1,4-diketopyrrolo[3,4-c]pyrrol,
- 3-(4'-Chlor-phenyl)-6-(4'-tert-butyl-phenyl)-2,5-dihydro-1,4-diketopyrrolo[3,4-c]pyrrol,
- 3-(4'-Chlor-phenyl)-6-(3',4'-dichlor-phenyl)-2,5-dihydro-1,4-diketopyrrolo[3,4-c]pyrrol,
- 3-(4'-Chlor-phenyl)-6-(3'-methyl-phenyl)-2,5-dihydro-1,4-diketopyrrolo[3,4-c]pyrrol und
- 3-(4'-Chlor-phenyl)-6-(3'-cyano-phenyl)-2,5-dihydro-1,4-diketopyrrolo[3,4-c]pyrrol.

Bevorzugt sind 2,5-Dihydro-1,4-diketopyrrolo[3,4-c]pyrrole der Formel (IIa), 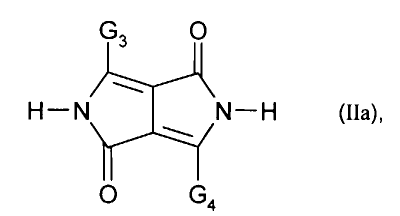 worin G₃ und G₄ unabhängig voneinander gleiche oder verschiedene Reste 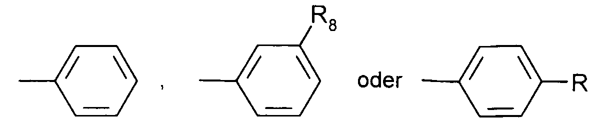 sind,
worin
- R₈: Fluor, Chlor, Cyano, Nitro, Trifluormethyl, C₁-C₄-Alkyl, C₁-C₄-Alkoxy, C₁-C₄-Alkylamino oder C₁-C₄-Dialkylamino bedeutet.

Besonders bevorzugt sind 2,5-Dihydro-1,4-diketopyrrolo[3,4-c]pyrrole der Formel (IIa), 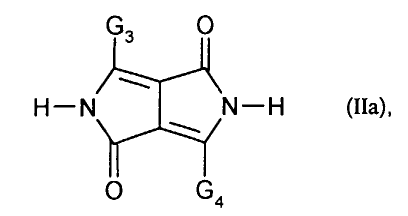 worin G₃ und G₄ unabhängig voneinander gleiche oder verschiedene Reste 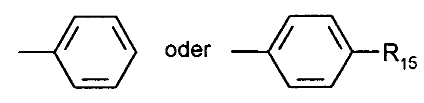 sind,
worin
- R₁₅: Chlor, Cyano, Nitro, Trifluormethyl, Methyl, Methoxy, Methylamino oder Dimethylamino bedeutet.

Besonders bevorzugt sind insbesondere die symmetrischen Verbindungen der Formel (IIa), worin G₃ und G₄ identisch sind.

Bevorzugt sind Chinacridone der Formel (IIb), 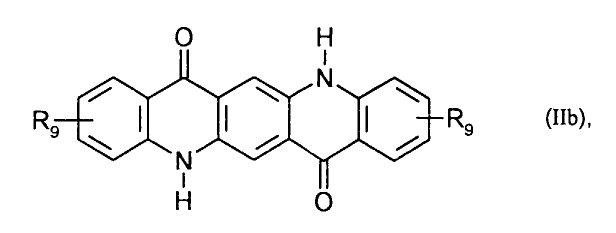 worin
- R₉: Wasserstoff, Halogen oder Methyl bedeutet.

Besonders bevorzugte Chinacridone der Formel (IIb) sind unsubstituiertes Chinacridon, 2,9-Dichlorchinacridon oder 2,9-Dimethylchinacridon.

Zweckmässig werden als Gast Verbindungen der Formeln (IIa) oder (IIb) eingesetzt, deren Raumanspruch kleiner ist, als derjenige des Wirtes der Formel (I). Als Mass für den Raumanspruch einer Verbindung kann man das Volumen deren Elementarzelle, welche durch Röntgenstrukturanalyse eines Monokristalls bestimmt werden kann, durch die Anzahl von Molekülen in der Elementarzelle dividieren. Gelingt dies nicht, zum Beispiel weil kein geeigneter Kristall zur Verfügung steht, so kann der Raumanspruch auch mit einem der vielen erhältlichen Computer-Simulationsprogrammen oder manuell anhand der üblichen, in Tabellenwerken wie "Handbook of Chemistry and Physics" (CRC press, 76^{th} edition, 1995/96, section 9) zu findenden Standardwerte für Bindungsabstände, Bindungswinkel und Van der Waals-Radien berechnet oder zumindest mit völlig genügender Genauigkeit abgeschätzt werden. Der Fachmann kann aber auch ohne jegliche Berechnung zum Beispiel leicht erkennen, dass 3,6-Di-(4-chlorphenyl)-2,5-dihydro-1,4-diketopyrrolopyrrol einen weit kleineren Raumanspruch besitzt als 3-(4-Biphenyl)-6-(4-tert.-butyl)-phenyl-2,5-dihydro-1,4-diketopyrrolopyrrol.

Bevorzugt betragen die molaren Konzentrationen 75-90 mol-% eines asymmetrischen 2,5-Dihydro-1,4-diketopyrrolo[3,4-c]pyrrols der Formel (I) und 25-10 mol-% eines 2,5-Dihydro-1,4-diketopyrrolo[3,4-c]pyrrols der Formel (IIa) oder eines Chinacridons der Formel(IIb).

Besonders bevorzugt betragen die molaren Konzentrationen 75-84 mol-% eines asymmetrischen 2,5-Dihydro-1,4-diketopyrrolo[3,4-c]pyrrols der Formel (I) und 25-16 mol-% eines 2,5-Dihydro-1,4-diketopyrrolo[3,4-c]pyrrols der Formel (IIa) oder eines Chinacridons der Formel (IIb).

Die 2,5-Dihydro-1,4-diketopyrrolo[3,4-c]pyrrole der Formeln (I) oder (IIa) und die Chinacridone der Formel (IIb) sind bekannte Substanzen. Sollten einige davon noch neu sein, so können sie in Analogie zu allgemein bekannten Methoden aus bekannten Substanzen hergestellt werden. Insbesondere können asymmetrische 2,5-Dihydro-1,4-diketopyrrolo[3,4-c]pyrrole der Formeln (I) oder (IIa) in reiner Form nach dem in US-4,778,899 beschriebenen Verfahren hergestellt werden.

Die erfindungsgemässen einphasigen festen Lösungen können ausgehend von physikalischen Mischungen der oben definierten Komponenten der Formeln (I) und (IIa) oder (IIb) nach folgenden an sich bekannten Verfahren hergestellt werden:
- durch Kontaktierung in polaren organischen Lösungsmitteln, bevorzugt durch Verrühren der Komponentenmischung bei Rückflusstemperatur,
- durch intensive Mahlung oder Knetung der Komponentenmischung,
- durch saure Umfällung, das heisst durch Auflösung der Komponentenmischung in Säure und Fällung der festen Lösung durch Verdünnen mit Wasser,
- durch alkalische Umfällung der Komponentenmischung in polaren organischen Lösungsmitteln,
- oder durch Verrühren der Komponentenmischung in polaren organischen Lösungsmitteln in Gegenwart von Alkalialkoholaten, Alkalihydroxiden oder quaternären Ammoniumverbindungen,
wobei in Analogie zu den Verfahren, wie sie zum Beispiel in US-4,783,540 detailliert beschrieben sind, vorgegangen werden kann.

Eine neue Herstellungsmethode besteht darin, dass
[1] die Verbindungen der Formeln (I) und (IIa) oder (IIb) nach an sich bekannten Methoden
   mit einem Dicarbonat der Formel

   L-O-L (III),

   oder mit einem Trihaloessigsäureester der Formel

   (R₁₆)₃C-L (IV),

   oder mit einem Azid der Formel

   L-N₃ (V),

   oder mit einem Carbonat der Formel

   L-OR₁₇ (VI),

   oder mit einem Alkylideniminooxyameisensäureester der Formel 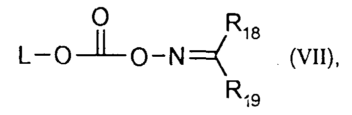 worin L eine Gruppe der Formel 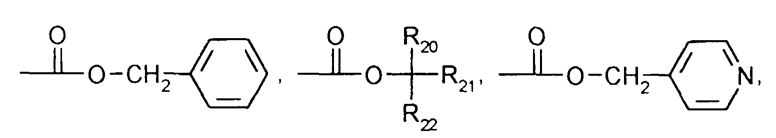 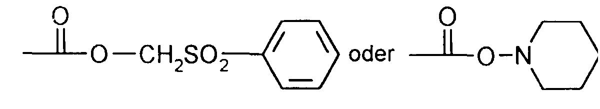 bedeutet,
   - R₁₆: Chlor, Fluor oder Brom,
   - R₁₇: C₁-C₄-Alkyl oder unsubstituiertes oder durch Halogen, C₁-C₄-Alkyl, C₁-C₄-Alkoxy oder -CN substituiertes Phenyl,
   - R₁₈: -CN oder -COOR₁₅,
   - R₁₉: unsubstituiertes oder durch Halogen, C₁-C₄-Alkyl, C₁-C₄-Alkoxy oder-CN substituiertes Phenyl,
   - R₂₀: Wasserstoff, C₁-C₆-Alkyl, C₂-C₅-Alkenyl oder C₂-C₅-Alkinyl, und
   - R₂₁ und R₂₂: unabhängig voneinander C₁-C₆-Alkyl, C₂-C₅-Alkenyl oder C₂-C₅-Alkinyl bedeuten,
   im Molverhältnis 1 : 2 bis 1 : 3 in einem aprotischen organischen Lösungsmittel in Gegenwart einer Base als Katalysator, in lösliche Pigmentvorläufer der Formeln 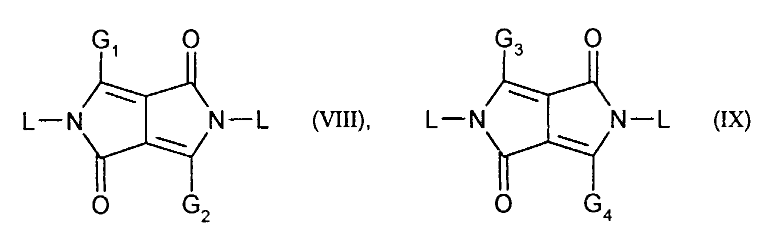 oder 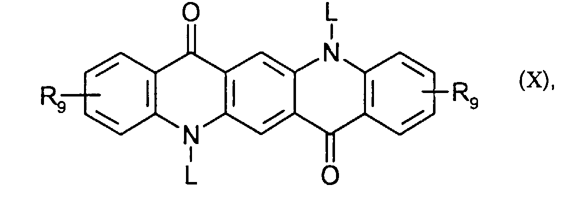 worin G₁ bis G₄ sowie R₉ die zuvor gegebene Bedeutung haben,
   umgesetzt werden; dann
[2] diese lösliche Pigmentvorläufer in den für die festen Lösungen gewünschten molaren Proportionen trocken oder als Suspension oder Lösung in einer inerten Flüssigkeit homogen vermischt werden; und anschliessend
[3] aus dem trockenen, suspendierten oder gelösten Gemisch durch thermische, photolytische oder chemische Behandlung oder Kombination derselben die gewünschte einphasige feste Lösung ausgefällt wird.

R₂₀, R₂₁ und R₂₂ bedeuten als C₂-C₅-Alkenyl zum Beispiel Vinyl, Allyl, Methallyl, n-But-2-enyl, 2-Methyl-prop-2-enyl oder n-Pent-2-enyl, und als C₂-C₅-Alkinyl zum Beispiel Ethinyl, Prop-2-inyl, But-2-inyl, But-3-inyl, 2-Methyl-but-3-inyl oder 2,2-Dimethyl-prop-2-inyl.

Bevorzugt sind R₂₀ sowie R₂₁ Methyl und R₂₂ C₁-C₆-Alkyl, insbesondere ebenfalls Methyl.

Bevorzugt ist L eine Gruppe der Formel

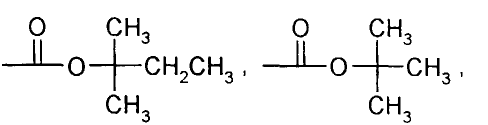

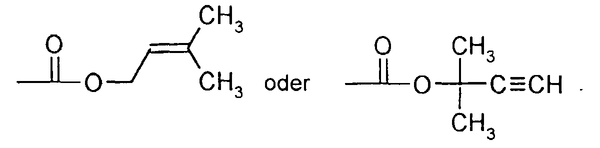

Bevorzugt werden die Verbindungen der Formeln (I), (IIa) und (IIb) zu den Verbindungen der Formeln (VIII), (IX) und (X) mit einem Dicarbonat der Formel (III) umgesetzt.

Die Dicarbonate der Formel (III), Trihaloessigsäureester der Formel (IV), Azide der Formel (V), Carbonate der Formel (VI) und Alkylideniminooxyameisensäureester der Formel (VII) sind bekannte Substanzen. Sollten einige noch neu sein, so können sie in Analogie zu allgemein bekannten Methoden hergestellt werden.

Geeignete aprotische organische Lösungsmittel sind beispielsweise Ether, wie Tetrahydrofuran oder Dioxan, oder Glykolether, wie Ethylenglykol-methylether, Ethylenglykol-ethylether, Diethylenglykol-monomethylether oder Diethylenglykol-monoethylether, ferner dipolar-aprotische Lösungsmittel, wie Acetonitril, Benzonitril, N,N-Dimethylformamid, N,N-Dimethylacetamid, Nitrobenzol, N-Methylpyrrolidon, halogenierte aliphatische Kohlenwasserstoffe, wie Dichlormethan, Trichlorethan, aromatische Kohlenwasserstoffe, wie Benzol oder durch Alkyl, Alkoxy oder Halogen substituiertes Benzol, wie Toluol, Xylol, Anisol oder Chlorbenzol, oder aromatische N-Heterocyclen, wie Pyridin, Picolin oder Chinolin. Bevorzugte Lösungsmittel sind Acetonitril, Dichlormethan, Tetrahydrofuran oder N,N-Dimethylformamid. Die genannten Lösungsmittel können auch als Mischungen eingesetzt werden. Zweckmässigerweise verwendet man 5-20 Gew.-Teile Lösungsmittel auf 1 Gew.-Teil der Reaktionsteilnehmer.

Als Katalysator geeignete Basen sind beispielsweise die Alkalimetalle selbst, wie Lithium, Natrium oder Kalium, sowie deren Hydroxide und Carbonate, oder Alkaliamide, wie Lithium-, Natrium- oder Kaliumamid, Alkalihydride, wie Lithium-, Natrium- oder Kaliumhydrid, oder Erdalkali- oder Alkalialkoholate, die sich insbesondere von primären, sekundären oder tertiären aliphatischen Alkoholen mit 1 bis 10 C-Atomen ableiten, wie z.B. Lithium-, Natrium- oder Kaliummethylat, -ethylat, -n-propylat, -isopropylat, -n-butylat, -sek.-butylat, -tert.-butylat, -2-methyl-2-butylat, -2-methyl-2-pentylat, -3-methyl-3-pentylat, -3-ethyl-3-pentylat, und ferner organische aliphatische, aromatische oder heterocyclische N-Basen, darunter z.B. Diazabicyclooctan, Diazabicycloundecen und 4-Dimethylaminopyridin, sowie Trialkylamine, wie z.B. Trimethyl- oder Triethylamin. Man kann auch ein Gemisch mehrerer der genannten Basen verwenden.

Bevorzugt sind die organischen N-Basen, wie z.B. Diazabicyclooctan, Diazabicycloundecen und insbesondere 4-Dimethylaminopyridin.

Die Umsetzung wird zweckmässig bei atmosphärischem Druck und Temperaturen zwischen 10°C und 100°C, insbesondere zwischen 14°C und 40°C, durchgeführt.

Die Verbindungen der Formeln (I) und (IIa) oder (IIb) werden danach in den gewünschten molaren Proportionen trocken oder als Suspension oder Lösung in einer inerten Flüssigkeit nach allgemein bekannten Methoden homogen vermischt.

Werden die Verbindungen der Formeln (I) und (IIa) oder (IIb) als Suspension oder Lösung in einer inerten Flüssigkeit vermischt, so können zweckmässig als inerte Flüssigkeit zum Beispiel folgende Lösungsmittel eingesetzt werden: Ether, wie Tetrahydrofuran oder Dioxan, oder Glykolether, wie Ethylenglykol-methylether, Ethylenglykol-ethylether, Diethylenglykol-monomethylether oder Diethylenglykol-monoethylether, Polyalkohole, wie Polyethylenglykol, Ketone, wie Aceton, Ethylmethylketon, Isobutylmethylketon oder Cyclohexanon, ferner dipolar-aprotische Lösungsmittel, wie Acetonitril, Benzonitril, N,N-Dimethylformamid, N,N-Dimethylacetamid, Nitrobenzol, N-Methylpyrrolidon, Dimethylsulfoxyd, halogenierte aliphatische Kohlenwasserstoffe, wie Trichlorethan, Dichlormethan oder Chloroform, aromatische Kohlenwasserstoffe, wie Benzol oder durch Alkyl, Alkoxy oder Halogen substituiertes Benzol, wie Toluol, Xylol, Anisol oder Chlorbenzol, aromatische N-Heterocyclen, wie Pyridin, Picolin oder Chinolin, hochsiedende aliphatische Kohlenwasserstoffe, wie Decalin, n-Dodecan oder Kerosin, oder Gemische derselben.

Bevorzugte Lösungsmittel sind Toluol, Xylol, Diphenylether, N-Methylpyrrolidon, N,N-Dimethylformamid, Dimethylsulfoxyd und Chinolin.

Die Konzentration der Verbindungen der Formeln (I) und (IIa) oder (IIb) in der inerten Flüssigkeit kann je nach verwendeter inerter Flüssigkeit stark variieren. Zweckmässig setzt man die Verbindung der Formeln (I) und (IIa) oder (IIb) in einer Konzentration von 0,1 bis 20 Gew.%, vorzugsweise 0,2 bis 5 Gew.%, bezogen auf die gesamte Lösung, ein.

Aus dem trockenen, suspendierten oder gelösten Gemisch der Pigmentvorläufer der Formeln (VIII) und (IX) oder (X) können die erfindungsgemässen einphasigen festen Lösungen bestehend aus den Verbindungen der Formeln (I) und (IIa) oder (IIb) auf einfachste Weise erhalten werden, sei es durch
a) thermische, d.h. z.B. durch Aufheizen auf Temperaturen zwischen 50°C und 400°C, bevorzugt zwischen 100°C und 200°C oder durch Laserbestrahlung,
b) photolytische, d.h. z.B. durch Belichtung mit Wellenlängen unter 375 nm, oder
c) chemische, d.h. z.B. mit organischen oder anorganischen Säuren, wie beispielsweise Essig-, Toluolsulfo-, Trifluoressig-, Salz- oder Schwefelsäure,

Behandlung des trockenen, suspendierten oder gelösten Gemisches und Isolierung des erhaltenen Produktes nach üblichen Methoden.

Die Behandlungsmethoden a), b) und c) können einzeln oder auch kombiniert verwendet werden. Bevorzugt sind die thermische Behandlung a) sowie die Kombination der thermischen Behandlung a) mit der chemischen Behandlung c).

Bevorzugt werden die erfindungsgemässen einphasigen festen Lösungen durch intensive Mahlung, alkalische Umfällung oder durch den oben beschriebenen Weg über die Pigmentvorläufer der Formeln (VIII) und (IX) oder (X) hergestellt. Besonders bevorzugt sind die alkalische Umfällung und insbesondere der Weg über die Pigmentvorläufer.

Das Röntgenbeugungsdiagramm der erfindungsgemässen einphasigen festen Lösungen ist wie bereits erwähnt durch das ausschliessliche Vorhandensein der Linien der asymmetrischen Komponente der Formel (I) gekennzeichnet. Leichte Abweichungen der Peaks des doppelten Glanzwinkels im Vergleich zum Röntgenbeugungsdiagramm des reinen Wirtes können insbesondere bei hoher Konzentration des Gastes beobachtet werden; sie betragen höchstens 0,3 2Θ im Durchschnitt der Absolutwerte für alle Peaks und bis zu etwa ±0,8 2Θ für einzelne Peaks. Solche Verschiebungen sind allerdings durch die Veränderung der chemischen Zusammensetzung bedingt und dürfen auf gar keinem Falle dahin interpretiert werden, dass die Kristallstruktur des Wirtes in irgend einer Weise verändert wäre. Die Halbwertsbreiten und die relativen Intensitäten der Linien des doppelten Glanzwinkels können ebenfalls leicht variieren. Bevorzugt sind erfindungsgemässe einphasige feste Lösungen mit hoher Kristallinität, charakterisiert durch Linien mit einer durchschnittlichen Halbwertsbreite ≤ 1,0 2Θ, besonders bevorzugt ≤ 0,8 2Θ, in deren Röntgenbeugungsdiagramm. Bevorzugt betragen die Absolutwerte der Abweichungen der Peaks des doppelten Glanzwinkels im Vergleich zum Röntgenbeugungsdiagramm des reinen Wirtes ≤ 0,2 2Θ im Durchschnitt aller Peaks und ≤ 0,4 2Θ bei einzelnen Peaks.

Durch die Bildung der erfindungsgemässen einphasigen festen Lösungen können sehr interessante und vorteilhafte koloristische Nuancenverschiebungen erzielt werden. Im Gegensatz zu den in der Einleitung beschriebenen, bekannten mehrphasigen festen Lösungen werden dabei überraschend Pigmente mit sehr guten Pigmenteigenschaften erhalten, insbesondere mit hoher Licht- und Wetterbeständigkeit sowie genau reproduzierbarer Nuance. Vorteilhaft ist ebenfalls, dass das Kristallgitter des asymmetrischen Wirtes im Gegensatz zum Kristallgitter symmetrischer Wirte überraschend im Wesentlichen erhalten bleibt, so dass der Wirt einfach nach dessen bekannten Eigenschaften, wie beispielsweise Oberflächenbeschaffenheit, hohe Echtheiten oder reine Farbnuance, ausgewählt werden kann. Der Gast dient unter anderem dazu, die Farbnuance der monophasigen festen Lösung auf die gewünschte Farbe einzustellen.

Die erfindungsgemässen einphasigen festen Lösungen können zur Optimierung ihrer Pigmenteigenschaften nach ihrer Entstehung noch zusätzlich thermisch behandelt oder rekristallisiert werden.

Die Rekristallisation bzw. thermische Behandlung geschieht gegebenenfalls nach für Pigmente üblichen Methoden. Im allgemeinen handelt es sich um eine thermische Nachbehandlung in Wasser oder in einem organischen Lösungsmittel, gegebenenfalls unter Druck. Vorzugsweise verwendet man organische Lösungsmittel, zum Beispiel durch Halogenatome, Alkyl- oder Nitrogruppen substituierte Benzole, wie Xylole, Chlorbenzol, o-Dichlorbenzol oder Nitrobenzol, sowie Pyridinbasen, wie Pyridin, Picolin oder Chinolin, ferner Ketone, wie Cyclohexanon, Alkohole, wie Isopropanol, Butanole oder Pentanole, Ether, wie Ethylenglykol-monomethyl- oder -monoethyl-ether, Amide, wie Dimethylformamid oder N-Methylpyrrolidon, oder Dimethylsulfoxid oder Sulfolan. Man kann die Nachbehandlung auch in Wasser, gegebenenfalls unter Druck, in Gegenwart von organischen Lösungsmitteln und/oder mit Zusatz von oberflächenaktiven Substanzen durchführen.

Die erfindungsgemässen festen Lösungen können als Pigmente zum Färben von hochmolekularem organischem Material verwendet werden.

Hochmolekulare organische Materialien, die mit den erfindungsgemässen einphasigen festen Lösungen gefärbt bzw. pigmentiert werden können, sind zum Beispiel Celluloseether und -ester, wie Ethylcellulose, Nitrocellulose, Celluloseacetat, Cellulosebutyrat, natürliche Harze oder Kunstharze, wie Polymerisationsharze oder Kondensationsharze, wie Aminoplaste, insbesondere Harnstoff- und Melamin-Formaldehydharze, Alkydharze, Phenoplaste, Polycarbonate, Polyolefine, Polystyrol, Polyvinylchlorid, Polyamide, Polyurethane, Polyester, ABS, Polyphenylenoxide, Gummi, Casein, Silikon und Silikonharze, einzeln oder in Mischungen.

Die erwähnten hochmolekularen organischen Verbindungen können einzeln oder in Gemischen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemässen einphasigen festen Lösungen als Toner oder in Form von Präparaten einzusetzen. Organische hochmolekulare Materialien, welche mit den erfindungsgemässen einphasigen festen Lösungen gefärbt sind, besitzen ausgezeichnete koloristische Eigenschaften. Gegenstand dieser Erfindung ist deshalb auch eine Stoffzusammensetzung, enthaltend eine erfindungsgemässe einphasige feste Lösung und ein hochmolekulares organisches Material.

Bezogen auf das zu pigmentierende hochmolekulare organische Material kann man die erfindungsgemässen einphasigen festen Lösungen in einer Menge von 0,01 bis 30 Gew.%, vorzugsweise von 0,1 bis 10 Gew. %, einsetzen.

Die Pigmentierung der hochmolekularen organischen Substanzen mit den erfindungsgemässen einphasigen festen Lösungen erfolgt beispielsweise derart, dass man solche feste Lösungen gegebenenfalls in Form von Masterbatches diesen Substraten unter Verwendung von Walzwerken, Misch- oder Mahlapparaten zumischt. Das pigmentierte Material wird hierauf nach an sich bekannten Verfahren, wie Kalandrieren, Pressen, Strangpressen, Streichen, Giessen oder Spritzgiessen, in die gewünschte endgültige Form gebracht. Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können zum Beispiel Ester der Phosphorsäure, Phthalsäure oder Sebacinsäure dienen. Die Weichmacher können vor oder nach der Einverleibung der erfindungsgemässen einphasigen festen Lösungen in die Polymere eingearbeitet werden. Zwecks Erzielung verschiedener Farbeffekte ist es ferner möglich, den hochmolekularen organischen Stoffen neben den erfindungsgemässen einphasigen festen Lösungen noch Füllstoffe, reflektierende metallische oder anorganische Partikel, wie beispielsweise Aluminiumflocken oder Glimmer, und/oder andere farbgebende Bestandteile, wie Weiss-, Bunt- oder Schwarzpigmente, in beliebigen Mengen zuzufügen.

Zum Pigmentieren von Lacken, Anstrichstoffen und Druckfarben werden die hochmolekularen organischen Materialien und die erfindungsgemässen einphasigen festen Lösungen gegebenenfalls zusammen mit Zusatzstoffen, wie Füllmitteln, anderen Pigmenten, Siccativen oder Weichmachern, in einem gemeinsamen organischen Lösungsmittel oder Lösungsmittelgemisch fein dispergiert bzw. gelöst. Man kann dabei so verfahren, dass man die einzelnen Komponenten einzeln oder auch mehrere gemeinsam dispergiert bzw. löst, und erst hierauf alle Komponenten zusammenbringt.

Besonders geeignet sind die erfindungsgemässen einphasigen festen Lösungen zum Einfärben von Kunststoffen, insbesondere Polyvinylchlorid und Polyolefinen, sowie zum Einfärben von Lacken, bevorzugt Automobil- oder Metalleffektlackierungen, beispielsweise solche, welche Metall- oder Mica-Partikel enthalten.

In Färbungen, beispielsweise von Polyvinylchlorid oder Polyolefinen, zeichnen sich die erfindungsgemässen einphasigen festen Lösungen durch gute allgemeine Pigmenteigenschaften, wie gute Dispergierbarkeit, hohe Farbstärke und Reinheit, gute Migrations-, Hitze-, Licht- und Wetterbeständigkeit sowie gute Deckkraft aus.

Die nachfolgenden Beispiele erläutern die Erfindung:

Beispiel 1: Eine Mischung aus 0,53 g (1,4 mMol) 2,9-Dichlorchinacridon, 2,12 g (5,6 mMol) 1,4-Diketo-3-(4-tert.-butyl-phenyl)-6-(4-chlor-phenyl)-2,5-dihydro-pyrrolo[3,4-c]pyrrol und 1,18 g Kaliumhydroxid in 40 ml Dimethylsulfoxid wird auf 50°C aufgewärmt und bei dieser Temperatur 2 Stunden gerührt. Danach wird das Reaktionsgemisch innert 1/4 Std einer Lösung von 0,7 ml konz. Schwefelsäure in 40 ml Methanol und 120 ml Wasser zugetropft, und das Gemisch wird unter Rührung während 5 Stunden auf 60°C erhitzt. Die rote Suspension wird abfiltriert und der Rückstand mit Methanol, dann Wasser gewaschen und im Vakuum bei 60°C getrocknet. Man erhält 2,3 g (87% d.Th.) eines roten Pulvers.

| Analyse | C | H | N | Cl |
|---|---|---|---|---|
| ber. | 68,40 % | 4,57 % | 7,38 % | 11,21 % |
| gef. | 67,14 % | 4,69 % | 7,15 % | 11,04 % |

Die vollständigen Röntgenbeugungsdiagramme werden nach üblichen Methoden mit Hilfe eines Siemens D500® Röntgen-Diffraktometers (CuK_{α}-Strahlung) bestimmt.

Das Röntgenbeugungsdiagramm ist durch folgende Beugungslinien gekennzeichnet:

| Netzebenenabstände [d-Werte in Å] | doppelte Glanzwinkel [2Θ] | relative Intensität [%] |
|---|---|---|
| 20,7870 | 4,25 | 100 |
| 6,5758 | 13,45 | 29 |
| 4,9359 | 17,96 | 88 |
| 3,6466 | 24,39 | 22 |
| 3,3364 | 26,70 | 85 |
| 3,0539 | 29,22 | 20 |

Dieses Röntgenbeugungsdiagramm ist praktisch identisch mit demjenigen von 1,4-Diketo-3-(4-tert.-butyl-phenyl)-6-(4-chlor-phenyl)-2,5-dihydro-pyrrolo[3,4-c]pyrrol, welches durch folgende Beugungslinien gekennzeichnet ist:

| Netzebenenabstände [d-Werte in Å] | doppelte Glanzwinkel [2Θ] | relative Intensität [%] |
|---|---|---|
| 20,3166 | 4,35 | 100 |
| 6,5391 | 13,53 | 22 |
| 4,9139 | 18,04 | 83 |
| 3,6357 | 24,46 | 15 |
| 3,3300 | 26,75 | 81 |
| 3,0498 | 29,26 | 17 |

Beispiel 2: Eine Mischung aus 0,86 g (3 mMol) 1,4-Diketo-3,6-diphenyl-2,5-dihydro-pyrrolo-[3,4-c]pyrrol, 2,36 g (7 mMol) 1,4-Diketo-3-(4-chlor-phenyl)-6-(4-methyl-phenyl)-2,5-dihydropyrrolo[3,4-c]pyrrol und 1,68 g Kaliumhydroxid in 60 ml Dimethylsulfoxid wird auf 50°C aufgewärmt und bei dieser Temperatur 2 Stunden gerührt. Danach wird das Reaktionsgemisch in eine Lösung von 0,9 ml konz. Schwefelsäure, 60 ml Methanol und 120 ml Wasser eingetragen und 6 Stunden bei 60°C gerührt. Die rote Suspension wird abfiltriert und der Rückstand mit Methanol, dann Wasser gewaschen und im Vakuum bei 60°C getrocknet. Man erhält 2,8 g (87% d.Th.) eines roten Pulvers.

| Analyse | C | H | N | Cl |
|---|---|---|---|---|
| ber. | 69,70 % | 3,97 % | 8,69 % | 7,70 % |
| gef. | 69,47 % | 3,98 % | 8,46 % | 7,61 % |

Das Röntgenbeugungsdiagramm ist durch folgende Beugungslinien gekennzeichnet:

| Netzebenenabstände [d-Werte in Å] | doppelte Glanzwinkel [2Θ] | relative Intensität [%] |
|---|---|---|
| 15,5890 | 5,67 | 83 |
| 7,6648 | 11,54 | 11 |
| 6,4784 | 13,66 | 22 |
| 5,9761 | 14,81 | 53 |
| 5,0935 | 17,40 | 12 |
| 4,9295 | 17,98 | 10 |
| 3,8330 | 23,19 | 27 |
| 3,7103 | 23,97 | 16 |
| 3,2959 | 27,03 | 100 |
| 3,1292 | 28,50 | 14 |
| 3,0635 | 29,13 | 12 |
| 2,9849 | 29,91 | 16 |
| 2,6988 | 33,17 | 9 |
| 2,1715 | 41,55 | 8 |

Dieses Röntgenbeugungsdiagramm ist praktisch identisch mit demjenigen von 1,4-Diketo-3-(4-chlor-phenyl)-6-(4-methyl-phenyl)-2,5-dihydro-pyrrolo[3,4-c]pyrrol, welches durch folgende Beugungslinien gekennzeichnet ist:

| Netzebenenabstände [d-Werte in Å] | doppelte Glanzwinkel [2Θ] | relative Intensität [%] |
|---|---|---|
| 15,3634 | 5,75 | 100 |
| 7,6399 | 11,57 | 23 |
| 6,4688 | 13,68 | 15 |
| 5,9654 | 14,84 | 35 |
| 5,0854 | 17,43 | 10 |
| 4,9062 | 18,07 | 7 |
| 3,8443 | 23,12 | 18 |
| 3,6949 | 24,07 | 10 |
| 3,2898 | 27,08 | 58 |
| 3,1260 | 28,53 | 10 |
| 3,0456 | 29,30 | 12 |
| 3,0085 | 29,67 | 14 |
| 2,6832 | 33,37 | 7 |
| 2,1752 | 41,48 | 10 |

Beispiel 3: Eine Mischung aus 0,72 g (2,5 mMol) 1,4-Diketo-3,6-diphenyl-2,5-dihydropyrrolo[3,4-c]pyrrol, 1,68 g (5,0 mMol) 1,4-Diketo-3-(4-chlor-phenyl)-6-(3-methyl-phenyl)-2,5-dihydro-pyrrolo[3,4-c]pyrrol und 0,66 g Natriumhydroxid in 75 ml Dimethylsulfoxid wird auf 50°C aufgewärmt und bei dieser Temperatur über Nacht gerührt. Danach wird das Reaktionsgemisch in eine auf 0°C gekühlte Lösung von 1,69 ml konz. Schwefelsäure in 150 ml Wasser eingetragen und 6 Stunden weitergerührt. Die rote Suspension wird abfiltriert und der Rückstand mit Methanol sowie Wasser gewaschen und im Vakuum bei 80°C getrocknet. Man erhält 1,6 g (67% d.Th.) eines roten Pulvers.

| Analyse | C | H | N | Cl |
|---|---|---|---|---|
| ber. | 70,17 % | 3,99 % | 8,79 % | 7,02 % |
| gef. | 69,45 % | 4,10 % | 8,68 % | 7,49 % |

Das Röntgenbeugungsdiagramm ist durch folgende Beugungslinien gekennzeichnet:

| Netzebenenabstände [d-Werte in Å] | doppelte Glanzwinkel [2Θ] | relative Intensität [%] |
|---|---|---|
| 15,2279 | 5,80 | 100 |
| - | ∼ 12 | < 10 |
| 6,6825 | 13,24 | 25 |
| 6,1163 | 14,47 | 73 |
| 4,9866 | 17,77 | 24 |
| 3,7645 | 23,62 | 29 |
| - | ∼ 25 | < 10 |
| 3,3240 | 26,80 | 94 |
| 3,0573 | 29,19 | 20 |

Dieses Röntgenbeugungsdiagramm ist praktisch identisch mit demjenigen von 1,4-Diketo-3-(4-chlor-phenyl)-6-(3-methyl-phenyl)-2,5-dihydro-pyrrolo[3,4-c]pyrrol, welches durch folgende Beugungslinien gekennzeichnet ist:

| Netzebenenabstände [d-Werte in Å] | doppelte Glanzwinkel [2Θ] | relative Intensität [%] |
|---|---|---|
| 15,2884 | 5,78 | 100 |
| 7,5655 | 11,69 | 13 |
| 6,8201 | 12,97 | 24 |
| 6,2102 | 14,25 | 54 |
| 5,0440 | 17,57 | 24 |
| 3,6942 | 24,07 | 22 |
| - | ∼ 25 | < 10 |
| 3,3300 | 26,75 | 80 |
| 3,1414 | 28,39 | 17 |

Beispiel 4: Man verfährt analog Beispiel 1, ersetzt jedoch 1,4 mMol 2,9-Dichlorchinacridon durch 1,4 mMol 2,9-Dimethylchinacridon. Man erhält ebenfalls ein rotes Pulver, dessen Röntgenbeugungsdiagramm mit demjenigen von 1,4-Diketo-3-(4-tert.-butyl-phenyl)-6-(4-chlor-phenyl)-2,5-dihydro-pyrrolo[3,4-c]pyrrol praktisch identisch ist.

Beispiel 5: Man verfährt analog Beispiel 2, setzt jedoch 3,3 mMol 1,4-Diketo-3,6-diphenyl-2,5-dihydro-pyrrolo[3,4-c]pyrrol und 6,7 mMol 1,4-Diketo-3-(4-chlor-phenyl)-6-(3-methylphenyl)-2,5-dihydro-pyrrolo[3,4-c]pyrrol ein. Man erhält ein rotes Pulver, dessen Röntgenbeugungsdiagramm mit demjenigen von 1,4-Diketo-3-(4-chlor-phenyl)-6-(3-methyl-phenyl)-2,5-dihydro-pyrrolo[3,4-c]pyrrol praktisch identisch ist.

Beispiel 6: Man verfährt analog Beispiel 2, setzt jedoch 4 mMol 1,4-Diketo-3,6-diphenyl-2,5-dihydro-pyrrolo[3,4-c]pyrrol und 6 mMol 1,4-Diketo-3-(4-chlor-phenyl)-6-(3-methyl-phenyl)-2,5-dihydro-pyrrolo[3,4-c]pyrrol ein. Man erhält ein rotes Pulver, dessen Röntgenbeugungsdiagramm mit demjenigen von 1,4-Diketo-3-(4-chlor-phenyl)-6-(3-methyl-phenyl)-2,5-dihydro-pyrrolo[3,4-c]pyrrol praktisch identisch ist.

Beispiel 7A: Einer Mischung von 14,75 g (51,2 mMol) 1,4-Diketo-3,6-diphenyl-2,5-dihydropyrrolo[3,4-c]pyrrol und 27,94 g (128 mMol) Di-ter.-butyl-dicarbonat in 500 ml Tetrahydrofuran werden 3,23 g (26,4 mMol) 4-Dimethylaminopyridin zugegeben. Die erhaltene rote Suspension wird 2 Stunden bei Raumtemperatur unter Ausschluss von Luftfeuchtigkeit gerührt. Das Lösungsmittel wird bei vermindertem Druck abdestilliert. Der gelbe Rückstand wird mit etwas Methanol gewaschen und im Vakuum bei Raumtemperatur getrocknet. Man erhält 23,8 g (95% d.Th.) gelbes N,N-Di-(tert.-butoxy-carbonyl)-1,4-diketo-3,6-diphenyl-2,5-dihydro-pyrrolo[3,4-c]pyrrol.

| Analyse | C | H | N |
|---|---|---|---|
| ber. | 68,84 % | 5,78 % | 5,73 % |
| gef. | 68,71 % | 5,79 % | 5,71 % |

Beispiel 7B: Man verfährt analog Beispiel 7A, ersetzt jedoch das 1,4-Diketo-3,6-diphenyl-2,5-dihydro-pyrrolo[3,4-c]pyrrol durch 17,23 g (51,2 mMol) 1,4-Diketo-3-(4-chlor-phenyl)-6-(3-methyl-phenyl)-2,5-dihydro-pyrrolo[3,4-c]pyrrol und verlängert die Reaktionszeit von 2 Stunden auf 30 Stunden. Man erhält 23,4 g (85% d.Th.) leuchtend gelbes N,N-Di-(tert.-butoxy-carbonyl)-1,4-diketo-3-(4-chlor-phenyl)-6-(3-methyl-phenyl)-2,5-dihydropyrrolo[3,4-c]pyrrol.

| Analyse | C | H | N | Cl |
|---|---|---|---|---|
| ber. | 64,86 % | 5,44 % | 5,22 % | 6,60 % |
| gef. | 64,50 % | 5,62 % | 5,11 % | 6,43 % |

Beispiel 7C: Eine Mischung von 1,95 g (4 mMol) N,N-Di-(tert.-butoxy-carbonyl)-1,4-diketo-3,6-diphenyl-2,5-dihydro-pyrrolo[3,4-c]pyrrol, hergestellt nach Beispiel 7A, und 4,30 g (8 mMol) N,N-Di-(tert.-butoxy-carbonyl)-1,4-diketo-3-(4-chlor-phenyl)-6-(3-methyl-phenyl)-2,5-dihydro-pyrrolo[3,4-c]pyrrol, hergestellt nach Beispiel 7B, wird unter Rühren in 200 ml Toluol bei 60°C gelöst. Nach Zugabe von 5,8 g Toluol-4-sulfonsäure wird kurz auf 105°C erhitzt, dann auf Raumtemperatur abkühlen gelassen. Die Suspension wird abfiltriert und der Rückstand zuerst mit Methanol, dann mit Wasser gewaschen und im Vakuum bei 80°C getrocknet. Man erhält 3,2 g (83% d.Th.) eines orangefarbenen Pulvers.

| Analyse | C | H | N | Cl |
|---|---|---|---|---|
| ber. | 70,17 % | 3,99 % | 8,79 % | 7,02 % |
| gef. | 69,17 % | 4,18 % | 8,51 % | 7,41 % |

Das Röntgenbeugungsdiagramm ist durch folgende Beugungslinien gekennzeichnet:

| Netzebenenabstände [d-Werte in Å] | doppelte Glanzwinkel [2Θ] | relative Intensität [%] |
|---|---|---|
| 15,0546 | 5,87 | 100 |
| 7,4994 | 11,79 | 9 |
| 6,7261 | 13,15 | 15 |
| 6,1222 | 14,46 | 48 |
| 4,9972 | 17,73 | 20 |
| 3,7475 | 23,72 | 17 |
| 3,5946 | 24,75 | 9 |
| 3,3249 | 26,79 | 68 |
| 3,0735 | 29,03 | 12 |

Dieses Röntgenbeugungsdiagramm ist praktisch identisch mit demjenigen von 1,4-Diketo-3-(4-chlor-phenyl)-6-(3-methyl-phenyl)-2,5-dihydro-pyrrolo[3,4-c]pyrrol.

Beispiel 8A: Man verfährt analog Beispiel 7B, ersetzt jedoch 1,4-Diketo-3-(4-chlor-phenyl)-6-(3-methyl-phenyl)-2,5-dihydro-pyrrolo[3,4-c]pyrrol durch 1,4-Diketo-3-(4-chlor-phenyl)-6-(4-methyl-phenyl)-2,5-dihydro-pyrrolo[3,4-c]pyrrol. Man erhält leuchtend gelbes N,N-Di-(tert.-butoxy-carbonyl)-1,4-diketo-3-(4-chlor-phenyl)-6-(4-methyl-phenyl)-2,5-dihydropyrrolo[3,4-c]pyrrol.

| Analyse | C | H | N | Cl |
|---|---|---|---|---|
| ber. | 64,86 % | 5,44 % | 5,22 % | 6,60 % |
| gef. | 64,35% | 5,70% | 5,10% | 6,45% |

Beispiel 8B: Man verfährt analog Beispiel 7C, ersetzt jedoch N,N-Di-(tert.-butoxy-carbonyl)-1,4-diketo-3-(4-chlor-phenyl)-6-(3-methyl-phenyl)-2,5-dihydro-pyrrolo[3,4-c]pyrrol durch N,N-Di-(tert.-butoxy-carbonyl)-1,4-diketo-3-(4-chlor-phenyl)-6-(4-methyl-phenyl)-2,5-dihydropyrrolo[3,4-c]pyrrol, hergestellt nach Beispiel 8A. Man erhält 3,1 g (80% d.Th.) eines roten Pulvers.

| Analyse | C | H | N | Cl |
|---|---|---|---|---|
| ber. | 70,17 % | 3,99 % | 8,79 % | 7,02 % |
| gef. | 69,17 % | 4,11 % | 8,60 % | 7,26 % |

Das Röntgenbeugungsdiagramm ist durch folgende Beugungslinien gekennzeichnet:

| Netzebenenabstände [d-Werte in Å] | doppelte Glanzwinkel [2Θ] | relative Intensität [%] |
|---|---|---|
| 15,1873 | 5,82 | 100 |
| 7,5993 | 11,64 | 12 |
| 6,4423 | 13,74 | 16 |
| 5,9374 | 14,91 | 48 |
| 5,0610 | 17,51 | 13 |
| 4,8989 | 18,09 | 9 |
| 3,8184 | 23,28 | 21 |
| 3,7018 | 24,02 | 12 |
| 3,2798 | 27,17 | 76 |
| 3,1160 | 28,63 | 10 |
| 3,0461 | 29,30 | 10 |
| 2,9827 | 29,93 | 14 |
| 2,6876 | 33,31 | 8 |
| 2,1658 | 41,67 | 8 |

Dieses Röntgenbeugungsdiagramm ist praktisch identisch mit demjenigen von 1,4-Diketo-3-(4-chlor-phenyl)-6-(4-methyl-phenyl)-2,5-dihydro-pyrrolo[3,4-c]pyrrol.

Beispiele 9-24: Man verfährt analog wie in Beispiel 2 beschrieben, verwendet jedoch als Gast eine Verbindung der Formel (IIa) (anstatt von 1,4-Diketo-3,6-diphenyl-2,5-dihydropyrrolo[3,4-c]pyrrol) und als Wirt eine Verbindung der Formel (I) (anstatt von 1,4-Diketo-3-(4-chlor-phenyl)-6-(4-methyl-phenyl)-2,5-dihydro-pyrrolo[3,4-c]pyrrol), wobei G₁ bis G₄ sowie das jeweilige Molverhältnis in der nachstehenden Tabelle angegeben sind. In allen Fällen erhält man einphasige feste Lösungen.

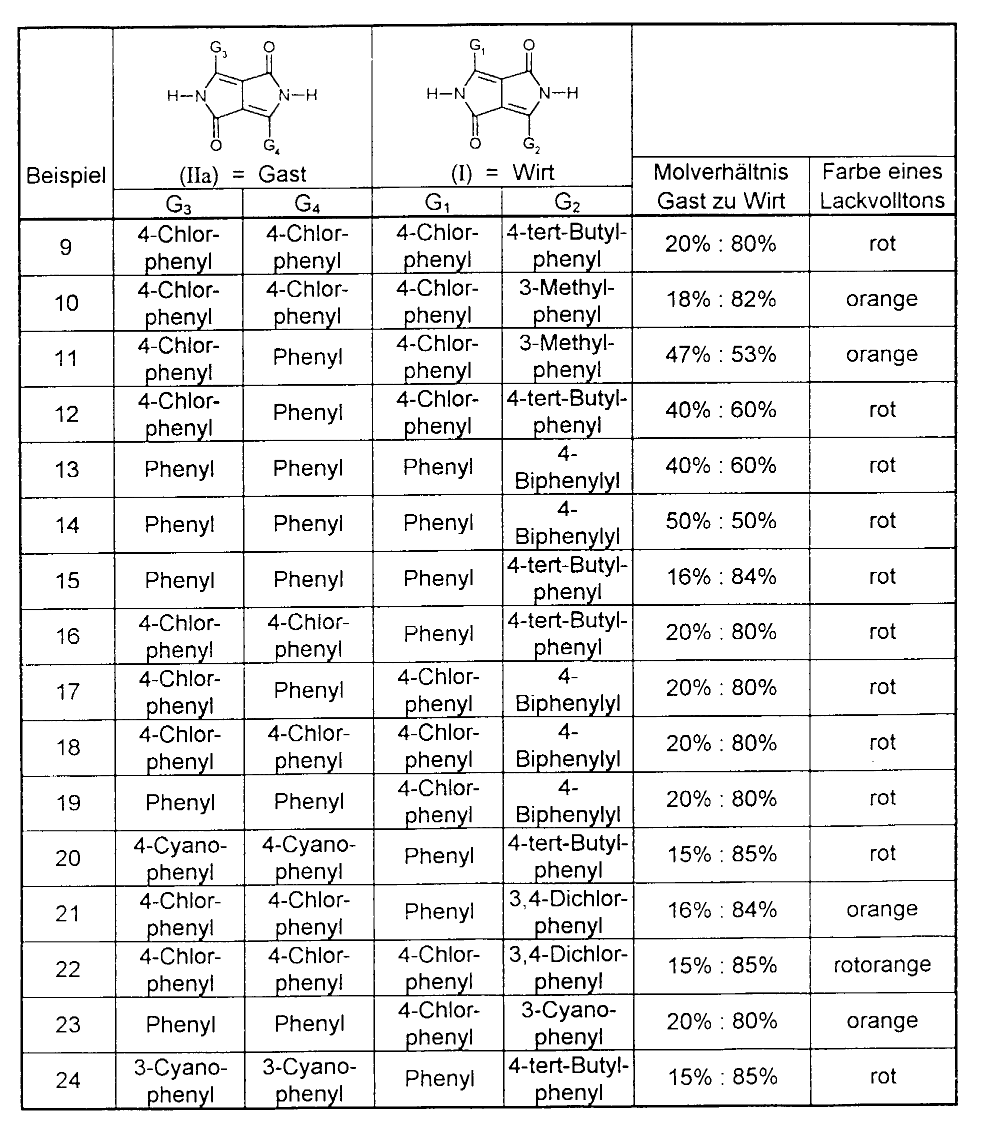

Beispiel 25: 7,5 g des Mischkristalls aus Beispiel 1, 98,9g CAB-Lösung bestehend aus

| | |
|---|---|
| 41,0 g | Celluloseacetobutyrat ®CAB 531.1, 20%ig in Butanol/Xylol 2:1 (Eastman Chem.) |
| 1,5 g | Zirkonium Octoat, |
| 18,5 g | ®SOLVESSO 150 (ESSO), |
| 21,5 g | Butylacetat und |
| 17,5 g | Xylol, |

36,5 g Polyesterharz ®DYNAPOL H700 (Dynamit Nobel), 4,6 g Melaminharz ®MAPRENAL MF650 (Hoechst) und 2,5 g Dispergiermittel ®DISPERBYK160 (Byk Chemie) werden zusammen während 90 Minuten mit einer Schüttelmaschine dispergiert (Total Lack 150g; 5% Pigment).

27,69 g des so erhaltenen Volltonlacks werden für die Base-coat-Lackierung mit 17,31 g Al-Stammlösung (8%ig) bestehend aus

| | |
|---|---|
| 12,65 g | ®SILBERLINE SS 3334AR, 60%ig (Silberline Ltd.) |
| 56,33 g | CAB Lösung (Zusammensetzung wie oben), |
| 20,81 g | Polyesterharz ®DYNAPOL H700, |
| 2,60 g | Melaminharz ®MAPRENAL MF650 und |
| 7,59 g | ®SOLVESSO 150, |

gemischt und auf ein Aluminiumblech spritzappliziert (Nassfilm ca. 20 µm). Nach einer Abdunstzeit von 30 Minuten bei Raumtemperatur wird ein TSA-Lack bestehend aus

| | |
|---|---|
| 29,60 g | Acrylharz ®URACRON 2263 XB, 50%ig in Xylol/Butanol (Chem. Fabrik Schweizerhalle), |
| 2,75 g | Butylglycolacetat, |
| 5,80 g | Melaminharz ®CYMEL 327, 90%ig in Isobutanol, |
| 5,70 g | Xylol, |
| 1,65 g | n-Butanol, |
| 0,50 g | Siliconöl, 1%ig in Xylol, |
| 3,00 g | Lichtschutzmittel ®TINUVIN 900, 10%ig in Xylol (Ciba) und |
| 1,00 g | Lichtschutzmittel ®TINUVIN 292, 10%ig in Xylol (Ciba), |

als Top-coat-Lackierung spritzappliziert (Nassfilm ca. 50 µm). Anschliessend wird der Lack nach weiteren 30 Minuten Abdunsten bei Raumtemperatur 30 Minuten bei 130°C eingebrannt.

Beispiel 26: 0,6 g der einphasigen festen Lösung aus Beispiel 2 werden mit 67 g Polyvinylchlorid, 33 g Dioctylphthalat, 2 g Dibutylzinndilaurat und 2 g Titandioxid vermischt und auf einem Walzenstuhl während 15 Minuten bei 160°C zu einer dünnen Folie verarbeitet. Die so erzeugte rote PVC-Folie ist sehr farbstark, migrations- und lichtbeständig.

Beispiel 27: 1000 g Polypropylengranulat (®DAPLEN PT-55, Chemie LINZ) und 20 g eines 50%-igen Pigmentpräparates, bestehend aus 10g der einphasigen festen Lösung von Beispiel 3 und 10 g Mg-Behenat, werden in einer Mischtrommel intensiv vermischt. Das so behandelte Granulat wird bei 260 bis 285°C nach dem Schmelzspinnverfahren versponnen. Man erhält orangegefärbte Fasern mit sehr guten Licht- und textilen Echtheiten.

Zum Färben von hochmolekularem organischem Material können analog zu den Beispielen 25-27 anstelle der Produkte aus den Beispielen 1-3 beispielsweise auch die Produkte der Beispiele 4-24 eingesetzt werden.

## Patentansprüche

1. Feste Lösung, bestehend aus 60-90 mol-% eines asymmetrischen 2,5-Dihydro-1,4-diketopyrrolo[3,4-c]pyrrols der Formel (I) 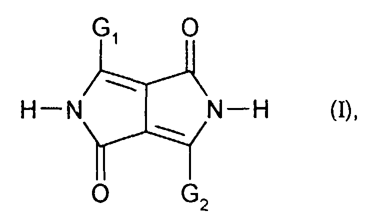 worin G₁ und G₂ unabhängig voneinander unterschiedliche Reste 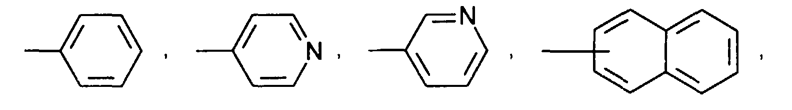 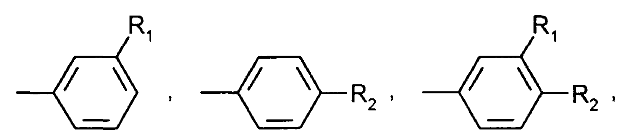 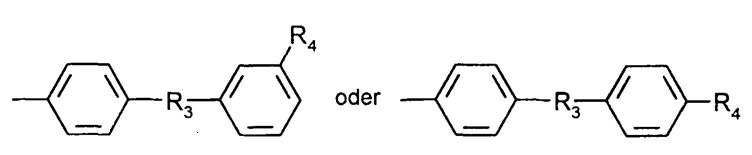 sind,
worin
R₁ und R₂ unabhängig voneinander Fluor, Chlor, Brom, Cyano, Nitro, Trifluormethyl, C₁-C₈-Alkyl, C₅C₆-Cycloalkyl, Phenyl, C₁-C₈-Alkoxy, C₁-C₈-Alkoxycarbonyl, C₁-C₈-Alkylamino, C₁-C₈-Alkylaminocarbonyl, C₁-C₈-Dialkylamino, C₁-C₈-Dialkylaminocarbonyl oder Morpholino,
R₃ -O-, -NR₅-, -N=N- oder -SO₂-,
R₄ Wasserstoff, Fluor, Chlor, Brom, Cyano, Nitro, Trifluormethyl, C₁-C₈-Alkyl, C₁-C₈-Alkoxy, C₁-C₈-Alkylamino, C₁-C₈-Dialkylamino, C₁-C₈-Alkoxycarbonyl, C₁-C₈-Alkylaminocarbonyl oder C₁-C₈-Dialkylaminocarbonyl, und
R₅ Wasserstoff, Methyl oder Ethyl bedeuten,
jedoch wenn einer der Reste G₁ oder G₂ 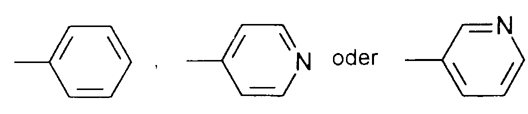 ist,
der jeweils andere Rest G₂ oder G₁ nicht 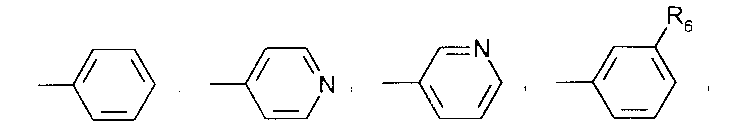 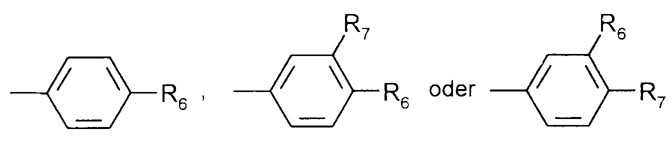 ist,
worin
R₆ Chlor und R₇ Fluor oder Trifluormethyl sind, oder R₆ sowie R₇ unabhängig voneinander Fluor, Methyl oder Trifluormethyl sind;
und 40-10 mol-% eines 2,5-Dihydro-1,4-diketopyrrolo[3,4-c]pyrrols der Formel (IIa) 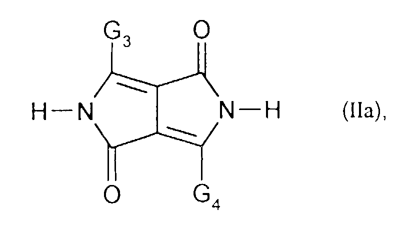 worin G₃ und G₄ unabhängig voneinander gleiche oder verschiedene Reste 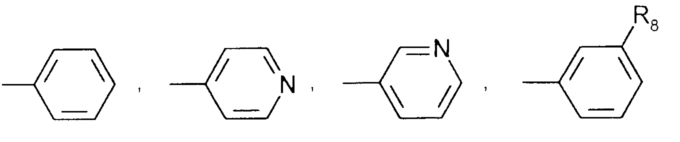 oder 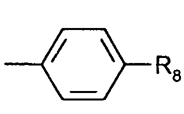 sind,
worin
R₈ Fluor, Chlor, Cyano, Nitro, Trifluormethyl, C₁-C₄-Alkyl, C₁-C₄-Alkoxy, C₁-C₄-Alkylamino oder C₁-C₄-Dialkylamino bedeutet;
oder 40-10 mol-% eines Chinacridons der Formel (IIb) 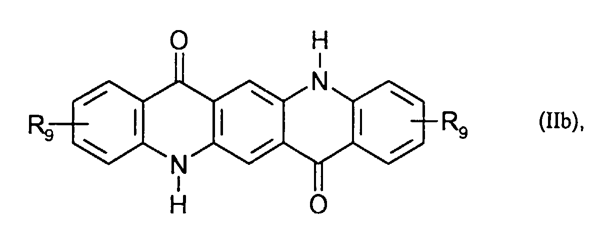 worin
R₉ Wasserstoff, Halogen, C₁-C₄-Alkyl oder C₁-C₄-Alkoxy bedeutet;
dadurch gekennzeichnet, dass es sich dabei um eine einphasige feste Lösung handelt, welche die Kristallstruktur des asymmetrischen 2,5-Dihydro-1,4-diketopyrrolo[3,4-c]pyrrols der Formel (I) aufweist.

2. Feste Lösung nach Anspruch 1, worin in Formel (I) G₁ und G₂ unabhängig voneinander unterschiedliche Reste 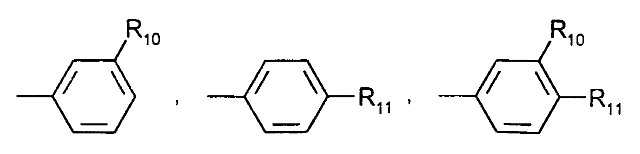 oder 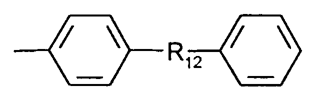 sind,
worin
R₁₀ und R₁₁ unabhängig voneinander Chlor, Brom, Cyano, Nitro, Trifluormethyl, C₁-C₈-Alkyl, Phenyl, C₁-C₈-Alkoxy, C₁-C₈-Alkoxycarbonyl, C₁-C₈-Alkylamino oder C₁-C₈-Dialkylamino sind,
und R₁₂ -NR₅- oder -SO₂- bedeutet.

3. Feste Lösung nach Anspruch 1, worin in Formel (I) G₁ ein Rest 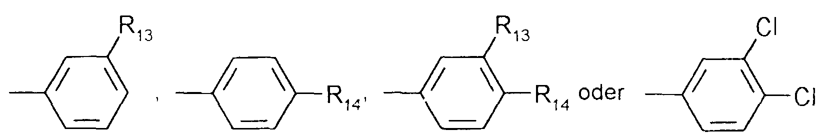 ist,
worin R₁₃ und R₁₄ Nitro, C₄-C₈-Alkyl, Phenyl, C₄-C₈-Alkoxy oder C₄-C₈-Dialkylamino, insbesondere tert.-Butyl oder Phenyl, bedeuten.

4. Feste Lösung nach Anspruch 1, worin in Formel (I) G₁ und G₂ unabhängig voneinander unterschiedliche Reste 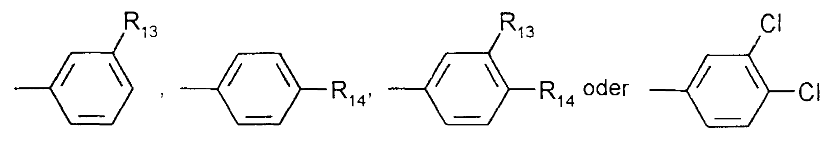 sind,
worin R₁₃ und R₁₄ Nitro, C₄-C₈-Alkyl, Phenyl, C₄-C₈-Alkoxy oder C₄-C₈-Dialkylamino, insbesondere tert.-Butyl oder Phenyl, bedeuten.

5. Feste Lösung nach Anspruch 1, worin in Formel (IIa) G₃ und G₄ unabhängig voneinander gleiche oder verschiedene Reste 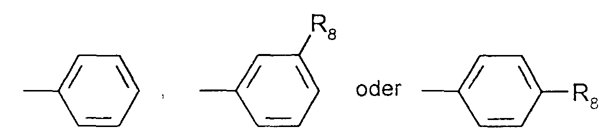 sind,
worin R₈ Fluor, Chlor, Cyano, Nitro, Trifluormethyl, C₁-C₄-Alkyl, C₁-C₄-Alkoxy, C₁-C₄-Alkylamino oder C₁-C₄-Dialkylamino bedeutet.

6. Feste Lösung nach Anspruch 1, worin in Formel (IIa) G₃ und G₄ unabhängig voneinander gleiche oder verschiedene Reste 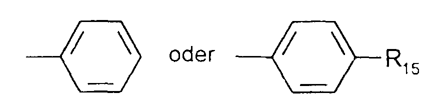 sind,
worin R₁₅ Chlor, Cyano, Nitro, Trifluormethyl, Methyl, Methoxy, Methylamino oder Dimethylamino bedeutet.

7. Feste Lösung nach Anspruch 1, worin in Formel (IIb) R₉ Wasserstoff, Halogen oder Methyl bedeutet.

8. Feste Lösung nach Anspruch 1, worin Formel (IIb) für unsubstituiertes Chinacridon, 2,9-Dichlorchinacridon oder 2,9-Dimethylchinacridon steht.

9. Feste Lösung nach Anspruch 1, worin die molaren Konzentrationen 75-90 mol-% eines asymmetrischen 2,5-Dihydro-1,4-diketopyrrolo[3,4-c]pyrrols der Formel (I) und 25-10 mol-% eines 2,5-Dihydro-1,4-diketopyrrolo[3,4-c]pyrrols der Formel (IIa) oder eines Chinacridons der Formel (IIb) betragen.

10. Feste Lösung nach Anspruch 1, worin die molaren Konzentrationen 75-84 mol-% eines asymmetrischen 2,5-Dihydro-1,4-diketopyrrolo[3,4-c]pyrrols der Formel (I) und 25-16 mol-% eines 2,5-Dihydro-1,4-diketopyrrolo[3,4-c]pyrrols der Formel (IIa) oder eines Chinacridons der Formel (IIb) betragen.

11. Feste Lösung nach Anspruch 1, welche nach ihrer Entstehung noch zusätzlich thermisch behandelt oder rekristallisiert wurde.

12. Verfahren zur Herstellung einer festen Lösung nach Anspruch 1, dadurch gekennzeichnet, dass die Komponentenmischung
- durch Kontaktierung in polaren organischen Lösungsmitteln, bevorzugt durch Verrühren bei Rückflusstemperatur,
- durch intensive Mahlung oder Knetung,
- durch saure Umfällung,
- durch alkalische Umfällung in polaren organischen Lösungsmitteln, oder
- durch Verrühren in polaren organischen Lösungsmitteln in Gegenwart von Alkalialkoholaten, Alkalihydroxiden oder quaternären Ammoniumverbindungen,
in die einphasige festen Lösung überführt wird.

13. Verfahren zur Herstellung einer festen Lösung nach Anspruch 1, dadurch gekennzeichnet, dass
[1] die Verbindungen der Formeln (I) und (IIa) oder (IIb) nach an sich bekannten Methoden
mit einem Dicarbonat der Formel
L-O-L (III),
oder mit einem Trihaloessigsäureester der Formel
(R₁₆)₃C-L (IV),
oder mit einem Azid der Formel
L-N₃ (V),
oder mit einem Carbonat der Formel
L-OR₁₇ (VI),
oder mit einem Alkylideniminooxyameisensäureester der Formel 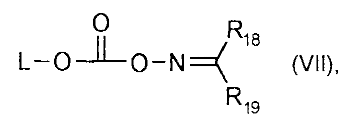 worin L eine Gruppe der Formel 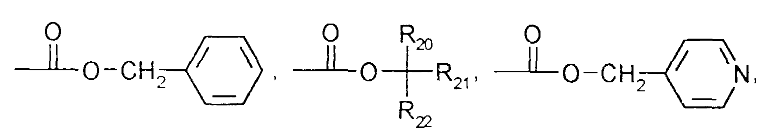 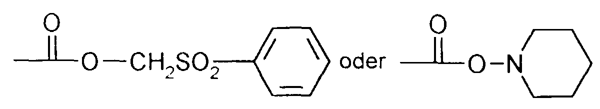 bedeutet,
R₁₆ Chlor, Fluor oder Brom,
R₁₇ C₁-C₄-Alkyl oder unsubstituiertes oder durch Halogen, C₁-C₄-Alkyl, C₁-C₄-Alkoxy oder -CN substituiertes Phenyl,
R₁₈ -CN oder -COOR₁₅,
R₁₉ unsubstituiertes oder durch Halogen, C₁-C₄-Alkyl, C₁-C₄-Alkoxy oder -CN substituiertes Phenyl,
R₂₀ Wasserstoff, C₁-C₆-Alkyl, C₂-C₅-Alkenyl oder C₂-C₅-Alkinyl, und
R₂₁ und R₂₂ unabhängig voneinander C₁-C₆-Alkyl, C₂-C₅-Alkenyl oder C₂-C₅-Alkinyl bedeuten,
im Molverhältnis 1 : 2 in einem aprotischen organischen Lösungsmittel in Gegenwart einer Base als Katalysator, in lösliche Pigmentvorläufer der Formeln 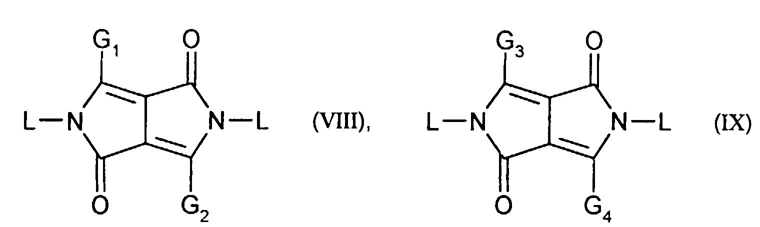 oder 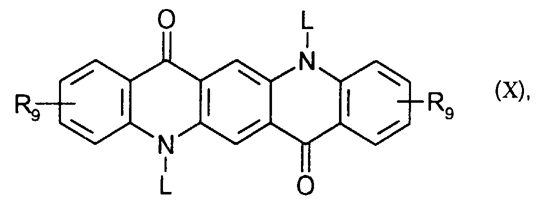 worin G₁ bis G₄ sowie R₉ die zuvor gegebene Bedeutung haben,
umgesetzt werden; dann
[2] diese lösliche Pigmentvorläufer in den für die festen Lösungen gewünschten molaren Proportionen trocken oder als Suspension oder Lösung in einer inerten Flüssigkeit homogen vermischt werden; und anschliessend
[3] aus dem trockenen, suspendierten oder gelösten Gemisch durch thermische, photolytische oder chemische Behandlung oder Kombination derselben die gewünschte einphasige feste Lösung ausgefällt wird.

14. Verfahren nach Anspruch 13, worin in Schritt [1] die Verbindungen der Formeln (I), (IIa) und (IIb) zu den Verbindungen der Formeln (VIII), (IX) und (X) mit einem Dicarbonat der Formel (III) umgesetzt werden.

15. Verfahren nach Anspruch 13, worin Schritt [3] thermisch, chemisch, oder durch Kombination von thermischer Behandlung mit chemischer Behandlung vollzogen wird.

16. Verwendung einer festen Lösung nach Anspruch 1 als Pigment zum Färben von hochmolekularem organischem Material.

17. Stoffzusammensetzung, enthaltend eine feste Lösung nach Anspruch 1 und ein hochmolekulares organisches Material.

## Claims

1. A solid solution consisting of 60-90 mol-% of an asymmetric 2,5-dihydro-1,4-diketopyrrolo[3,4-c]pyrrole of the formula (I) 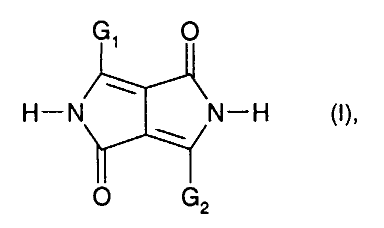 in which G₁ and G₂ independently of one another are different radicals 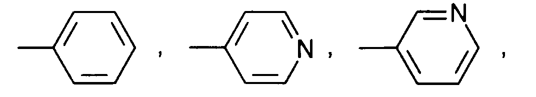 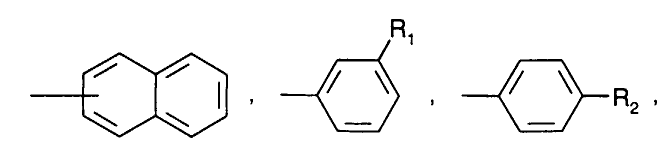 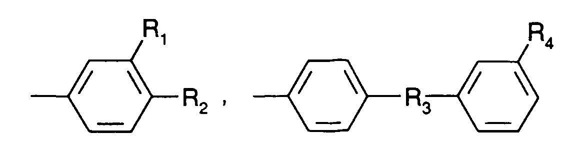 or 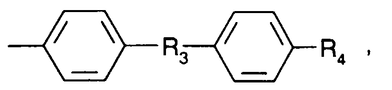 where
R₁ and R₂ independently of one another are fluorine, chlorine, bromine, cyano, nitro, trifluoromethyl, C₁-C₈alkyl, C₅-C₆cycloalkyl, phenyl, C₁-C₈alkoxy, C₁-C₈alkoxycarbonyl, C₁-C₈alkylamino, C₁-C₈alkylaminocarbonyl, C₁-C₈dialkylamino, C₁-C₈dialkylaminocarbonyl or morpholino,
R₃ is -O-, -NR₅-, -N=N- or -SO₂-,
R₄ is hydrogen, fluorine, chlorine, bromine, cyano, nitro, trifluoromethyl, C₁-C₈alkyl, C₁-C₈alkoxy, C₁-C₈alkylamino, C₁-C₈dialkylamino, C₁-C₈alkoxycarbonyl, C₁-C₈alkylaminocarbonyl or C₁-C₈dialkylaminocarbonyl, and
R₅ is hydrogen, methyl or ethyl,
with the proviso that, if one of the radicals, G₁ or G₂, is 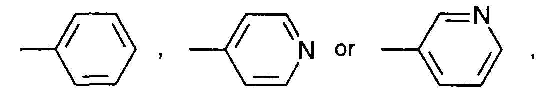 the other radical, G₂ or G₁, is not 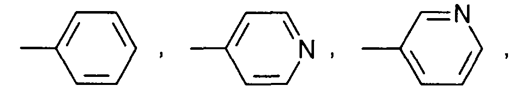 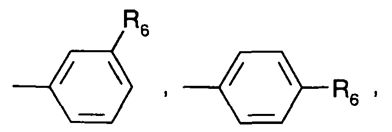 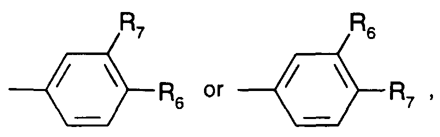 where
R₆ is chlorine and R₇ is fluorine or trifluoromethyl, or
R₆ and R₇ independently of one another are fluorine, methyl or trifluoromethyl;
and 40-10 mol-% of a 2,5-dihydro-1,4-diketopyrrolo[3,4-c]pyrrole of the formula (IIa) 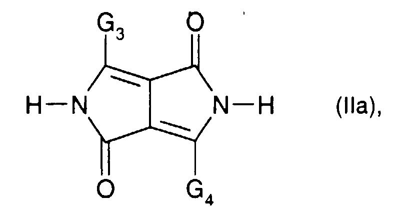 in which G₃ and G₄ independently of one another are identical or different radicals 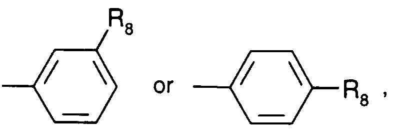 where
R₈ is fluorine, chlorine, cyano, nitro, trifluoromethyl, C₁-C₄alkyl, C₁-C₄alkoxy, C₁-C₄alkylamino or C₁-C₄dialkylamino;
or 40-10 mol-% of a quinacridone of the formula (llb) 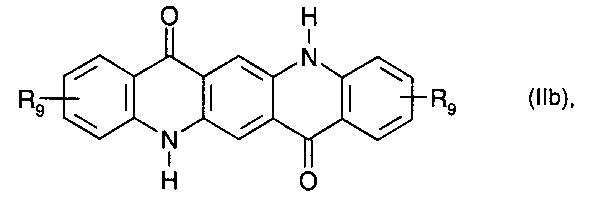 in which
R₉ is hydrogen, halogen, C₁-C₄alkyl or C₁-C₄alkoxy;
which is a single-phase solid solution having the crystal structure of the asymmetric 2,5-dihydro-1,4-diketopyrrolo[3,4-c]pyrrole of the formula (I).

2. A solid solution according to claim 1, wherein in formula (I) G₁ and G₂ independently of one another are different radicals 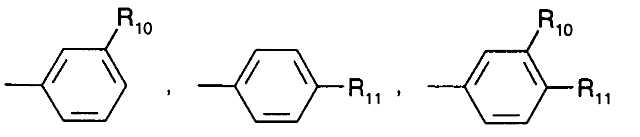 or 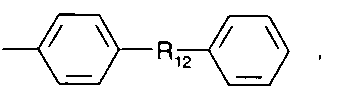 where
R₁₀ and R₁₁ independently of one another are chlorine, bromine, cyano, nitro, trifluoromethyl, C₁-C₈alkyl, phenyl, C₁-C₈alkoxy, C₁-C₈alkoxycarbonyl, C₁-C₈alkylamino or C₁-C₈dialkylamino,
and R₁₂ is -NR₅- or -SO₂-.

3. A solid solution according to claim 1, wherein in formula (I) G₁ is a radical 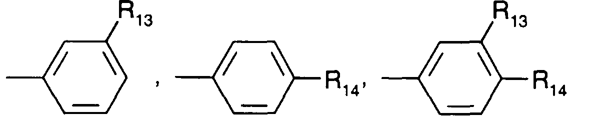 or 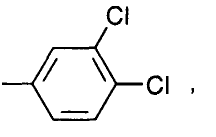 in which R₁₃ and R₁₄ are nitro, C₄-C₈alkyl, phenyl, C₄-C₈alkoxy or C₄-C₈dialkylamino, in particular tert-butyl or phenyl

4. A solid solution according to claim 1, wherein in formula (I) G₁ and G₂ independently of one another are different radicals 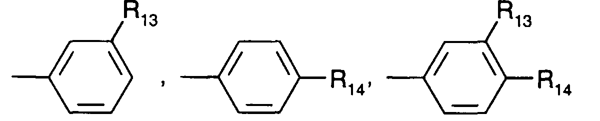 or 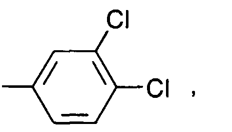 in which R₁₃ and R₁₄ are nitro, C₄-C₈alkyl, phenyl, C₄-C₈alkoxy or C₄-C₈dialkylamino, in particular tert-butyl or phenyl.

5. A solid solution according to claim 1, wherein in formula (IIa) G₃ and G₄ independently of one another are identical or different radicals 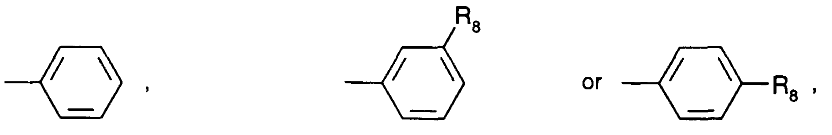 in which R₈ is fluorine, chlorine, cyano, nitro, trifluoromethyl, C₁-C₄alkyl, C₁-C₄alkoxy, C₁-C₄alkylamino or C₁-C₄dialkylamino.

6. A solid solution according to claim 1, wherein in formula (IIa) G₃ and G₄ independently of one another are identical or different radicals 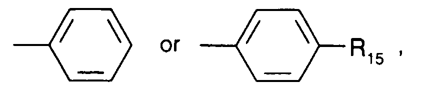 in which R₁₅ is chlorine, cyano, nitro, trifluoromethyl, methyl, methoxy, methylamino or dimethylamino.

7. A solid solution according to claim 1, wherein in formula (IIb) R₉ is hydrogen, halogen or methyl.

8. A solid solution according to claim 1, wherein formula (IIb) is unsubstituted quinacridone, 2,9-dichloroquinacridone or 2,9-dimethylquinacridone.

9. A solid solution according to claim 1, wherein the molar concentrations are 75-90 mol-% of an asymmetric 2,5-dihydro-1,4-diketopyrrolo[3,4-c]pyrrole of the formula (I) and 25-10 mol-% of a 2,5-dihydro-1,4-diketopyrrolo[3,4-c]pyrrole of the formula (IIa) or of a quinacridone of the formula (IIb).

10. A solid solution according to claim 1, wherein the molar concentrations are 75-84 mol-% of an asymmetric 2,5-dihydro-1,4-diketopyrrolo[3,4-c]pyrrole of the formula (I) and 25-16 mol-% of a 2,5-dihydro-1,4-diketopyrrolo[3,4-c]pyrrole of the formula (IIa) or of a quinacridone of the formula (IIb).

11. A solid solution according to claim 1, which after its formation has been additionally subjected to thermal treatment or to recrystallization.

12. A process for preparing a solid solution according to claim 1, which comprises converting the mixture of components into the single-phase solid solution
- by contacting in polar organic solvents, preferably by stirring together at reflux temperature,
- by intensive grinding or kneading,
- by acidic reprecipitation,
- by alkaline reprecipitation in polar organic solvents, or
- by stirring together in polar organic solvents in the presence of alkali metal alcoholates, alkali metal hydroxides or quaternary ammonium compounds.

13. A process for preparing a solid solution according to claim 1, which comprises
[1] reacting the compounds of the formulae (I) and (lla) or (llb) by methods known per se with a dicarbonate of the formula
L-O-L (III),
or with a trihaloacetate of the formula
(R₁₆)₃C-L (IV),
or with an azide of the formula
L-N₃ (V),
or with a carbonate of the formula
L-OR₁₇ (VI),
or with an alkylideneiminooxyformate of the formula 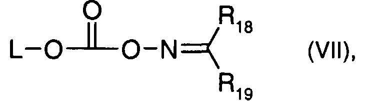 in which L is a group of the formula 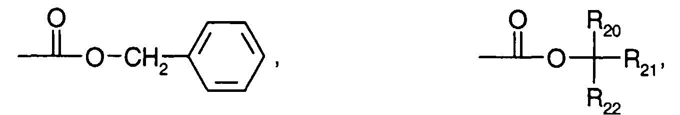 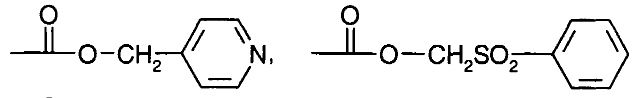 or 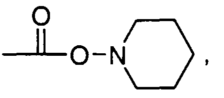
R₁₆ is chlorine, fluorine or bromine,
R₁₇ is C₁-C₄alkyl or phenyl or halogen-, C₁-C₄alkyl-, C₁-C₄alkoxy- or -CN-substituted phenyl,
R₁₈ is -CN or -COOR₁₅,
R₁₉ is phenyl or halogen-, C₁-C₄alkyl-, C₁-C₄alkoxy- or -CN-substituted phenyl,
R₂₀ is hydrogen, C₁-C₆alkyl, C₂-C₅alkenyl or C₂-C₅alkynyl, and
R₂₁ and R₂₂ independently of one another are C₁-C₆alkyl, C₂-C₅alkenyl or C₂-C₅alkynyl,
in a molar ratio of 1 : 2 in an aprotic organic solvent in the presence of a base as catalyst, to give soluble pigment precursors of the formula 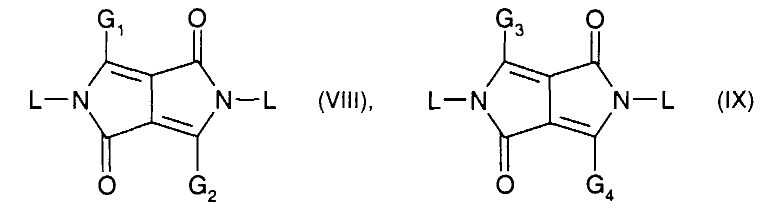 or 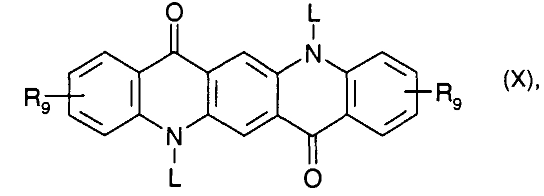 in which G₁ to G₄ and R₉ are as defined above,
[2] homogeneously mixing these soluble pigment precursors in an inert liquid, in the molar proportions desired for the solid solutions, and in dry form or as a suspension or solution; and then
[3] precipitating the desired single-phase solid solution from the dry, suspended or dissolved mixture by thermal, photolytic or chemical treatment or a combination thereof.

14. A process according to claim 13, wherein in step [1] the compounds of the formulae (I), (IIa) and (IIb) are reacted with a dicarbonate of the formula (III) to give the compounds of the formula (VIII), (IX) and (X).

15. A process according to claim 13, wherein step [3] is accomplished thermally, chemically or by combination of thermal treatment with chemical treatment.

16. The use of a solid solution according to claim 1 as a pigment for colouring high molecular mass organic material.

17. A composition comprising a solid solution according to claim 1 and a high molecular mass organic material.

## Revendications

1. Solution solide constituée par 60 à 90 % molaires d'un 2,5-dihydro-1,4-dicétopyrrolo[3,4-c]-pyrrole asymétrique de formule (I) 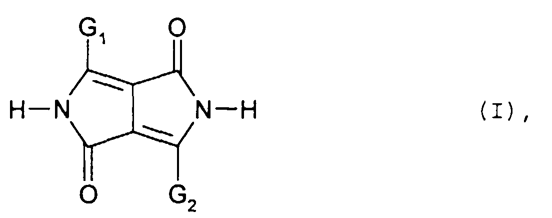 où
G₁ et G₂ représentent, indépendamment l'un de l'autre, des restes différents 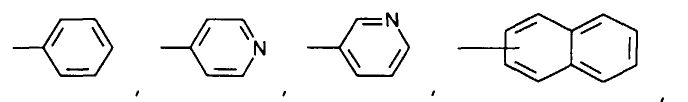 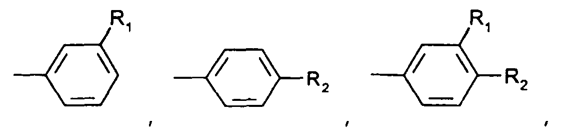 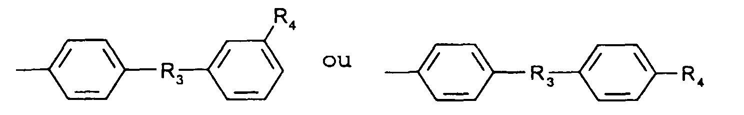 où
R₁ et R₂ représentent, indépendamment l'un de l'autre, des atomes de fluor, de chlore, de brome, des groupes cyano, nitro, trifluorométhyle, alkyle en C₁-C₈, cycloalkyle en C₅-C₆, phényle, alkoxy en C₁-C₈, (alkoxy en C₁-C₈)carbonyle, (alkyl en C₁-C₈)amino, (alkyl en C₁-C₈)aminocarbonyle, di(alkyl en C₁-C₈)-amino, di(alkyl en C₁-C₈)-aminocarbonyle ou morpholino,
R₃ représente des groupes -O-, -NR₅-, -N=N- ou -SO₂-,
R₄ représente des atomes d'hydrogène, de fluor, de chlore, de brome, des groupes cyano, nitro, trifluorométhyle, alkyle en C₁-C₈, alkoxy en C₁-C₈, (alkyl en C₁-C₈)amino, di(alkyl en C₁-C₈)amino, (alkoxy en C₁-C₈)carbonyle, (alkyl en C₁-C₈)-aminocarbonyle, di(alkyl en C₁-C₈)-aminocarbonyle et
R₅ représente un atome d'hydrogène, des méthyle ou éthyle,
toutefois, si un des restes G₁ ou G₂ représente 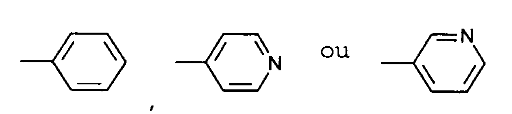 l'autre reste G₂ ou G₁ ne représente pas 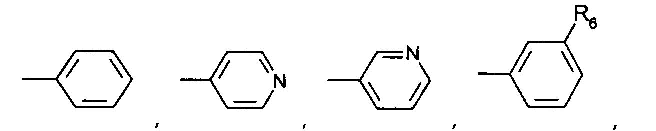 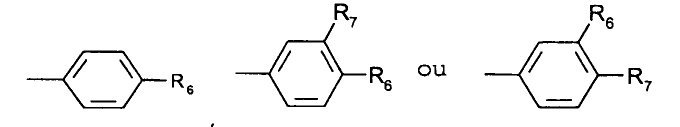 où
R₆ représente un atome de chlore et R₇ représente un atome de fluor ou un groupe trifluorométhyle , ou
R₆ ainsi que R₇ représentent, indépendamment l'un de l'autre, un atome de fluor, des groupes méthyle ou trifluorométhyle ;
et de 40 à 10 % molaires d'un 2,5-dihydro-1,4-dicétopyrrolo[3,4-c]pyrrole de formule (IIa) 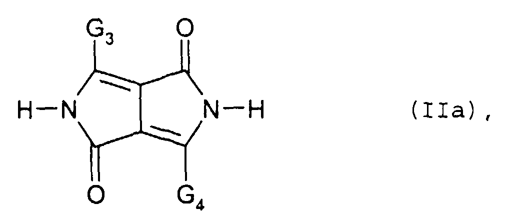 où
G₃ et G₄ représentent, indépendamment l'un de l'autre, des restes identiques ou différents 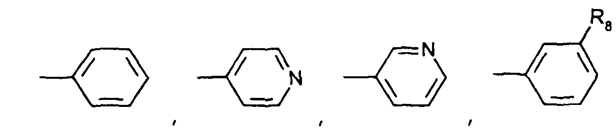 ou 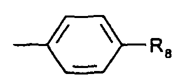 où
R₈ représente des atomes de fluor, de chlore, des groupes cyano, nitro, trifluorométhyle, alkyle en C₁-C₄, alkoxy en C₁-C₄, (alkyl en C₁-C₄)amino ou di(alkyl en C₁-C₄)-amino ;
ou de 40 à 10 % molaires d'une quinacridone de formule (IIb) 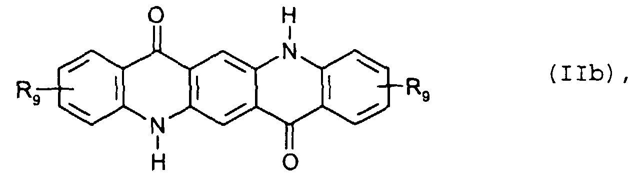 où
R₉ un atome d'hydrogène, un atome d'halogène, des groupes alkyle en C₁-C₄, alkoxy en C₁-C₄ ;
caractérisé en ce qu'il s'agit d'une solution solide monophasique qui présente la structure cristalline du 2,5-dihydro-1,4-dicétopyrrolo[3,4-c]pyrrole asymétrique de formule (I).

2. Solution solide selon la revendication 1, où dans la formule (I) G₁ et G₂ représentent, indépendamment l'un de l'autre, des restes différents 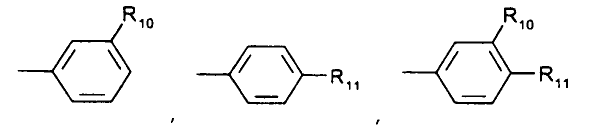 ou 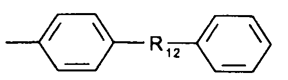 où
R₁₀ et R₁₁ représentent, indépendamment l'un de l'autre, des atomes de chlore, de brome, des groupes cyano, nitro, trifluorométhyle, alkyle en C₁-C₈, phényle, alkoxy en C₁-C₈, (alkoxy en C₁-C₈)-carbonyle, (alkyl en C₁-C₈)-amino ou di(alkyl en C₁-C₈)amino,
et
R₁₂ représente des groupes -NR₅- ou -SO₂-.

3. Solution solide selon la revendication 1, où dans la formule (I) G₁ représente un reste 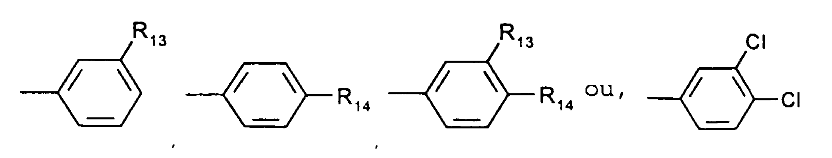 où R₁₃ et R₁₄ représentent des groupes nitro, alkyle en C₄-C₈, phényle, alkoxy en C₄-C₈, ou di(alkyl en C₄-C₈)amino, en particulier tert.-butyle ou phényle.

4. Solution solide selon la revendication 1, où dans la formule (I) G₁ et G₂ représentent, indépendamment l'un de l'autre, 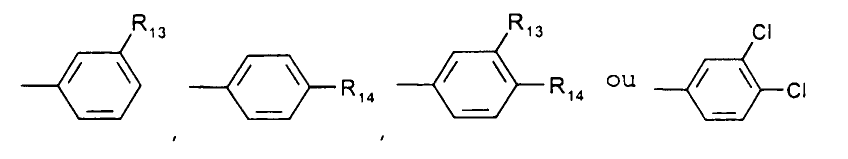 où R₁₃ et R₁₄ représentent des groupes nitro, alkyle en C₄-C₈, phényle, alkoxy en C₄-C₈, ou di(alkyl en C₄-C₈)amino, en particulier tert.-butyle ou phényle.

5. Solution solide selon la revendication 1, où dans la formule (IIa) G₃ et G₄ représentent, indépendamment l'un de l'autre, des restes identiques ou différents 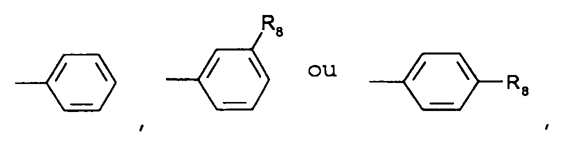 où R₈ représente des atomes de fluor, de chlore, des groupes cyano, nitro, trifluorométhyle, alkyle en C₁-C₄, alkoxy en C₁-C₄, (alkyl en C₁-C₄)amino ou di(alkyl en C₁-C₄)amino.

6. Solution solide selon la revendication 1, où dans la formule (IIa) G₃ et G₄ représentent, indépendamment l'un de l'autre, des restes identiques ou différents 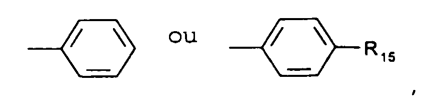 où R₁₅ représente un atome de chlore, des groupes cyano, nitro, trifluorométhyle, méthyle, méthoxy, méthylamino ou diméthylamino.

7. Solution solide selon la revendication 1, où dans la formule (IIb) R₂₂ représente un atome d'hydrogène, un atome d'halogène ou un groupe méthyle.

8. Solution solide selon la revendication 1, où la formule (IIb) représente la quinacridone non substituée, la 2,9-dichloroquinacridone ou la 2,9diméthyl-quinacridone.

9. Solution solide selon la revendication 1, où les concentrations molaires s'élèvent de 75 à 90 % molaires d'un 2,5-dihydro-1,4-dicétopyrrolo[3,4-c]-pyrrole asymétrique de formule (I) et de 25 à 10 % molaires d'un 2,5-dihydro-1,4-dicétopyrrolo [3,4-c]-pyrrole de formule (IIa) ou d'une quinacridone de formule (IIb).

10. Solution solide selon la revendication 1, où les concentrations molaires vont de 75 à 84 % molaires d'un 2,5-dihydro-1,4-dicétopyrrolo[3,4-c]pyrrole asymétrique de formule (I) et de 25 à 16 % molaires d'un 2,5-dihydro-1,4-dicétopyrrolo[3,4-c]pyrrole de formule (IIa) ou d'une quinacridone de formule (IIb).

11. Solution solide selon la revendication 1, que l'on traite encore, après sa formation, par la chaleur ou par recristallisation.

12. Procédé pour la préparation d'une solution solide selon la revendication 1, caractérisé en ce qu'on transforme le mélange de constituants
- par contact dans des solvants organiques polaires, de préférence par mélange sous agitation à la température de reflux,
- par broyage ou malaxage intense,
- par précipitation acide,
- par précipitation alcaline dans des solvants organiques polaires, ou
- par mélange sous agitation dans des solvants organiques polaires en présence d'alcoolates alcalins, d'hydroxydes alcalins ou de composés d'ammonium quaternaires,
en la solution solide monophasique.

13. Procédé pour la préparation d'une solution solide selon la revendication 1, caractérisé en ce que
[1] on fait réagir les composés de formules (I) et (IIa) ou (IIb) selon des méthodes connues en soi, sur
un dicarbonate de formule
L-O-L (III),
ou un trihaloacétate de formule
(R₁₆)₃C-L (IV),
ou un azoture de formule
L-N₃ (V),
ou un carbonate de formule
L-OR₁₇ (VI),
ou un alkylidène imino-oxyformiate de formule 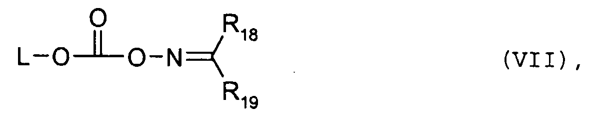 où L représente un groupe de formule 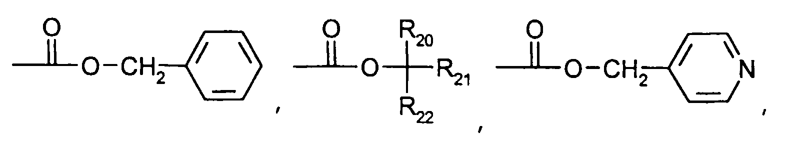 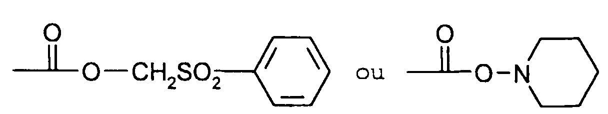
R₁₆ représente des atomes de chlore, de fluor ou de brome,
R₁₇ représente un groupe alkyle en C₁-C₄ ou phényle non substitué ou substitué par des atomes d'halogène, des groupes alkyle en C₁-C₄, alkoxy en C₁-C₄ ou -CN,
R₁₈ représente des groupes -CN ou -COOR₁₅,
R₁₉ représente un groupe phényle non substitué ou substitué par des atomes d'halogènes, alkyle en C₁-C₄, alkoxy en C₁-C₄ ou -CN,
R₂₀ représente un atome d'hydrogène, des groupes alkyle en C₁-C₆, alcényle en C₂-C₅ ou alcynyle en C₂-C₅, et
R₂₁ et R₂₂ représentent, indépendamment l'un de l'autre, des groupes alkyle en C₁-C₆, alcényle en C₂-C₅ ou alcynyle en C₂-C₅,
dans un rapport molaire 1:2 dans un solvant aprotique organique en présence d'une base en tant que catalyseur, pour obtenir des précurseurs solubles de pigments de formules 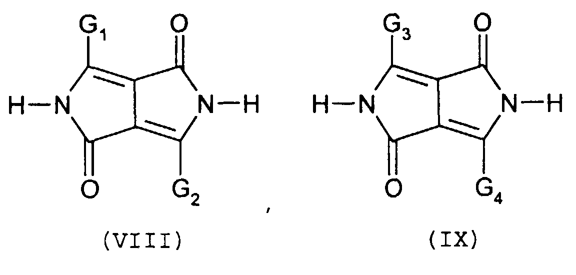 ou 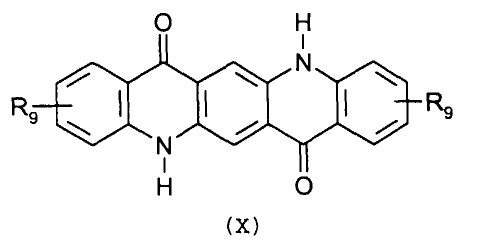 où
G₁ à G₄ ainsi que R₉ possèdent les significations données auparavant, puis
[2] on mélange ces précurseurs solubles de pigments dans des proportions molaires souhaitées pour les solutions solides à sec ou sous forme de suspension ou de solution dans un liquide inerte pour obtenir un mélange homogène ; et ensuite
[3] on précipite à partir du mélange sec, suspendu ou dissous la solution solide monophasique voulue au moyen d'un traitement thermique, photolytique ou chimique ou d'une combinaison de ces traitements.

14. Procédé selon la revendication 13, où dans le stade [1], on transforme les composés de formules (I), (IIa) et (IIb) en composés de formules (VIII), (IX) et (X) en employant un dicarbonate de formule (III).

15. Procédé selon la revendication 13, où dans le stade [3], la réaction est terminée en appliquant un traitement thermique, chimique ou une combinaison de traitement thermique et de traitement chimique.

16. Utilisation d'une solution solide selon la revendication 1 en tant que pigment pour la teinture de matière organique de haut poids moléculaire.

17. Composition de matières contenant une solution solide selon la revendication 1 et une matière organique de haut poids moléculaire.
